# EUROPÄISCHE PATENTSCHRIFT

(11) **EP 1 379 312 B1**
(45) Veröffentlichungstag und Bekanntmachung des Hinweises auf die Patenterteilung: **02.11.2005**
(21) Anmeldenummer: 01943260.8
(22) Anmeldetag: 18.04.2001
(51) Int. Cl.: A61N 5/06

(54) **BESTRAHLUNGSVORRICHTUNG INSBESONDERE ZUR PHOTODYNAMISCHEN DIAGNOSE ODER THERAPIE**
IRRADIATION DEVICE, PARTICULARLY FOR CARRYING OUT PHOTODYNAMIC DIAGNOSIS OR THERAPY
DISPOSITIF D'IRRADIATION, ADAPTE EN PARTICULIER A LA THERAPIE OU AU DIAGNOSTIC PHOTODYNAMIQUE

(43) Veröffentlichungstag der Anmeldung: 14.01.2004
(73) Patentinhaber: Edel, Susann, 82031 Grünwald (DE); Knott, Georg, 82377 Penzberg (DE)
(72) Erfinder: Edel, Susann, 82031 Grünwald (DE); Knott, Georg, 82377 Penzberg (DE)
(74) Vertreter: Banzer, Hans-Jörg
(86) Internationale Anmeldenummer: PCT/EP2001/004409
(87) Internationale Veröffentlichungsnummer: WO 2002/083237

(56) Entgegenhaltungen:
- WO-A-93/09847
- WO-A-93/21842
- WO-A-99/19024
- CH-A- 651 477
- DE-A- 4 108 328
- DE-A- 19 832 904
- DE-C- 19 943 393
- US-B1- 6 214 033
- PATENT ABSTRACTS OF JAPAN vol. 1997, no. 05, 30. Mai 1997 (1997-05-30) & JP 09 010238 A (OSADA RES INST LTD), 14. Januar 1997 (1997-01-14)

## Beschreibung

Die vorliegende Erfindung betrifft eine Bestrahlungsvorrichtung zur Erzielung einer möglichst homogenen Bestrahlung mit einer möglichst hohen Bestrahlungsstärke für unterschiedliche Anwendungen, insbesondere für medizinische, kosmetische oder industrielle Anwendungen.

In der Medizin haben sich in den letzten Jahren die sog. Photodynamische Diagnose (PDD) und Photodynamische Therapie (PDT) zu einer wichtigen Alternative bei der Erkennung und Behandlung neoplastischer Veränderungen eines Patienten entwickelt. Beide Verfahren beruhen auf der Absorption von Licht geeigneter Wellenlänge durch eine lichtempfindliche Substanz, dem sog. Photosensibilisator. Wird eine möglichst selektive Anreicherung des Photosensibilisators im neoplastischen Gewebe des Patienten erzielt, können bei der Photodynamischen Diagnose durch Auswertung des in Folge der Bestrahlung des Photosensibilisators emittierten Fluoreszenzlichts neoplastische Veränderungen, wie beispielsweise Tumore, relativ zuverlässig erkannt werden. Bei der Wahl des Photosensibilisators ist eine hohe Tumorselektivität des Photosensibilisators auf der einen Seite sowie eine hohe Lichtabsorption und Quantenausbeute auf der anderen Seite wichtig. Bei der Photodynamischen Therapie wird hingegen die Tatsache ausgenutzt, dass es zwischen dem Photosensibilisator und der biologischen Umgebung über eine komplexe Wechselwirkung zur Aktivierung von molekularem Sauerstoff in dem jeweils behandelten Gewebe kommt, wobei die auf diese Weise erzeugten Sauerstoffradikale hoch reaktiv sind und Biomoleküle in nächster Umgebung letal schädigen können. Auf diese Weise kann ebenfalls durch Verabreichung eines Photosensibilisators mit anschließender Bestrahlung ein therapeutischer Nutzen, wie z. B. die Behandlung von gutartigen oder bösartigen Tumoren, Gewebeläsionen und/oder Warzen etc., erzielt werden.

Für die oben genannten medizinischen Photodynamischen Bestrahlungsanwendungen sind bisher Bestrahlungsvorrichtungen bekannt, welche polychromatische Bestrahlungsquellen mit hochenergetischen Lampen aufweisen, wobei diese Bestrahlungsvorrichtungen beispielsweise zur Ganzkörperbestrahlung oder zur Bestrahlung einzelner Körperregionen, wie z. B. auch eines Bereichs der Mundhöhle, eingesetzt werden können. Bei diesen herkömmlichen Bestrahlungsvorrichtungen ist neben dem relativ komplexen Aufbau auch die Verwendung der zuvor erwähnten hochenergetischen Lampen problematisch, da diese hochenergetischen Lampen einerseits eine relativ aufwendige Ansteuerung mit entsprechend hoher Stromaufnahme benötigen und andererseits Wärmestrahlung mit breitbandigem Licht erzeugen, welche für den Patienten unangenehm oder bei falscher Anwendung sogar schmerzhaft sein kann.

Für andere Anwendungszwecke sind zwar bereits Bestrahlungsvorrichtungen bekannt, bei denen als Leuchtmittel beispielsweise Leuchtdioden (LED) in Form eines Arrays oder einer Matrix in einer Ebene angeordnet sind (sog. LED-Cluster). Leuchtdioden weisen jedoch in der Regel eine sehr geringe Bestrahlungsstärke auf, welche zu einer intensiven Bestrahlung, die bei der zuvor erwähnten Photodynamischen Diagnose und Photodynamischen Therapie erforderlich ist, nicht ausreicht. Derartige Bestrahlungsvorrichtungen, welche Leuchtdioden in herkömmlicher Art nebeneinander eben angeordnet aufweisen, sind daher für das zuvor beschriebene medizinische Anwendungsgebiet der Photodynamischen Diagnose- und Therapieanwendung nicht geeignet.

Der vorliegenden Erfindung liegt die Aufgabe zugrunde, eine Bestrahlungsvorrichtung bereitzustellen, welche mit möglichst einfachen Mitteln die Bestrahlung einer gewünschten Bestrahlungsfläche mit einer relativ hohen Bestrahlungsstärke ermöglicht, wobei insbesondere eine größtmögliche Homogenität der Bestrahlung der gewünschten Bestrahlungsfläche gewährleistet sein soll. Die Bestrahlungsvorrichtung soll darüber hinaus insbesondere für den Einsatz in einer medizinischen Photodynamischen Diagnose- oder Therapieanwendung geeignet sein.

Die oben genannte Aufgabe wird erfindungsgemäß durch eine Bestrahlungsvorrichtung mit den Merkmalen des Anspruchs 1 gelöst. Die Unteransprüche definieren jeweils bevorzugte und vorteilhafte Ausführungsformen bzw. Anwendungen der vorliegenden Erfindung.

Die erfindungsgemäße Bestrahlungsvorrichtung umfasst mindestens zwei flächige Leuchtsegmente, welche jeweils eine gerichtete Strahlung erzeugen, wobei die flächigen Leuchtsegmente derart winkelig zueinander angeordnet sind, dass sich die einzelnen gerichteten Strahlungen auf einer Bestrahlungsfläche, welche sich in einem bestimmten Abstand zu der Bestrahlungsvorrichtung befindet, im Wesentlichen vollständig bzw. exakt überlappen. Auf diese Weise kann eine gegenüber herkömmlichen Bestrahlungsvorrichtungen vielfach höhere Bestrahlungsstärke (mW/cm²) erzielt werden, welche eine intensive Bestrahlung in Allgemein-, Medizin-, Kosmetik- und Industrieanwendungen ermöglicht.

Bestrahlungseinrichtungen mit mehreren winkelig zueinander angeordneten Flächigen Leuchtsegmenten sind bekannt aus WO 99/19024, WO 93/09847 und DE 4 108 328. Diese Bestrahlungseinrichtungen sind gemäß dem Oberbegriff des Anspruchs 1.

Als Leuchtsegmente werden dabei bevorzugt regelmäßige Anordnungen von Leuchtmitteln, insbesondere Leuchtdioden oder Laserdioden, verwendet, welche z.B. UV-Strahlung, IR-Strahlung oder sichtbares Licht emittieren und die gewünschte gerichtete Strahlung des jeweiligen Leuchtsegments dadurch erzeugen, dass der Abstrahlwinkel der einzelnen Leuchtmittel auf einen bestimmten Wert, ≤ 30° beschränkt ist.

Die im Rahmen der vorliegenden Erfindung vorgeschlagene Anordnung von Leuchtmitteln, welche normalerweise eine relativ geringe Bestrahlungsstärke aufweisen, in Form der zuvor erwähnten Leuchtsegmente und die spezielle geometrische Anordnung der einzelnen Leuchtsegmente zueinander ermöglicht die Erzielung einer gegenüber dem Stand der Technik vielfach höheren Bestrahlungsstärke, wobei die einzelnen Leuchtmittel eine relativ geringe Energieaufnahme aufweisen und keine aufwendige Ansteuerung voraussetzen. Durch die Verwendung von Leuchtsegmenten mit einer Vielzahl von gleichmäßig angeordneten Leuchtmitteln, welche matrixartig in Spalten und Reihen oder auch in zueinander versetzten Reihen angeordnet sein können, ist zudem ein größtmögliches Maß an Homogenität der Bestrahlung gewährleistet, da die sich ergebende exakte optische Abbildung der einzelnen Leuchtsegmente auf der gewünschten Bestrahlungsfläche zu einer optimalen Bestrahlungsstärkeverteilung führen.

Wie bereits zuvor beschrieben worden ist, können als Leuchtmittel bevorzugt handelsübliche Leuchtdioden zum Einsatz kommen, wobei bevorzugt Leuchtdioden mit einem relativ kleinen Abstrahlwinkel und einer relativ hohen Bestrahlungsstärke verwendet werden. Die Auswahl der jeweils verwendeten Leuchtmittel wird hinsichtlich der abgestrahlten spektralen Hauptwellenlängen von der in der jeweiligen Bestrahlungsvorrichtung benötigten effektiven Bestrahlungswellenlänge bestimmt. So können beispielsweise Leuchtdioden mit einer Wellenlänge im Bereich von 630 nm, insbesondere im Bereich von 635 nm, verwendet werden, wenn bei einer medizinischen Photodynamischen Therapieanwendung der gewünschte medizinische Effekt dieser Therapie im Bereich dieser Wellenlänge auftritt. Bei einer medizinischen Photodynamischen Diagnoseanwendung können Wellenlängen im Bereich 370 nm - 430 nm erforderlich sein, wenn die zur Diagnose notwendige Anregung beispielsweise bei den Wellenlängen 370 nm und 428 nm auftritt. Bei einer kosmetischen Anwendung, wie beispielsweise einer Haarbleichung, können Leuchtmittel verwendet werden, deren emittierte Wellenlänge sich im Bereich von 500 nm, insbesondere 502 nm, befindet, wenn der kosmetische Effekt der Haarbleichung in der Nähe dieser Wellenlänge auftritt. Um für unterschiedliche Anwendungen nicht mehrere derartige Bestrahlungsvorrichtungen verwenden zu müssen, ist es vorteilhaft, wenn die einzelnen Leuchtsegmente einer Bestrahlungsvorrichtung unterschiedliche Gruppen von Leuchtmitteln aufweisen, wobei jede Leuchtmittelgruppe in dem jeweiligen Leuchtsegment regelmäßig verteilt angeordnet ist und die einzelnen Leuchtmittelgruppen unterschiedliche Wellenlängen emittieren, so dass in Abhängigkeit von der jeweils gewünschten Anwendung lediglich die entsprechende Leuchtmittelgruppe aktiviert bzw. eingeschaltet werden muss, wobei weiterhin auf der gewünschten Bestrahlungsfläche eine hohe Bestrahlungsstärke mit homogener Bestrahlung erzielt werden kann.

Die auf der gewünschten Bestrahlungsfläche erzeugte Vervielfachung der Bestrahlungsstärke ist abhängig von der Anzahl und der Form der einzelnen Leuchtsegmente, da sich jedes Leuchtsegment optisch auf die Bestrahlungsfläche abbildet. Die Grundfläche der einzelnen Leuchtsegmente wird dabei derart gewählt, dass sich die von den einzelnen Leuchtsegmenten erzeugten Strahlungen auf der gewünschten Bestrahlungsfläche mit einer bestimmten Form überlappen. Weisen die einzelnen Leuchtsegmente eine rechteckige Form auf, ist die Form der Überlappung der einzelnen Strahlungen ebenfalls rechteckig. Ebenso können mehreckige oder abgerundete Formen der einzelnen Leuchtsegmente gewählt werden, wobei die Grundfläche der einzelnen Leuchtsegmente nicht identisch sein muss. Bei der Wahl der Grundfläche der einzelnen Leuchtsegmente ist vielmehr zu berücksichtigen, dass sich aufgrund det winkeligen Anordnung der einzelnen Leuchtsegmente zueinander und der daraus resultierenden unterschiedlichen Ausrichtung der einzelnen Leuchtsegmente zu der gewünschten Bestrahlungsfläche unterschiedliche Strahlungsabbildungen der einzelnen Leuchtsegmente auf der gewünschten Bestrahlungsfläche ergeben, d.h. die Form eines senkrecht zu der Bestrahlungsfläche gegenüber liegenden Leuchtsegments wird unverändert abgebildet, während die Form eines zu der Bestrahlungsfläche unter einem relativ spitzen Winkel angeordneten Leuchtsegments verzerrt abgebildet wird. Soll auf der Bestrahlungsfläche beispielsweise eine kreisförmige Überlappung der Strahlungen der einzelnen Leuchtsegmente erzielt werden, kann es daher erforderlich sein, die zu der Bestrahlungsfläche unter einem relativ spitzen Winkel angeordneten Leuchtsegmente mit einer elliptischen Form auszubilden, während die zu der Bestrahlungsfläche unter einem weniger spitzen Winkel bzw. rechtwinkelig angeordneten Leuchtsegmente mit einer kreisrunden Form ausgebildet sein können, so dass sich auf der gewünschten Bestrahlungsfläche jeweils kreisförmige Strahlformen überlappen.

Die einzelnen Leuchtsegmente bzw. Leuchtsegmentträger können aneinander angrenzend bzw. zueinander benachbart angeordnet und in einem gemeinsamen Gehäuse untergebracht sein. Insbesondere zur Bestrahlung von einzelnen Körperregionen eines Lebewesens bzw. eines Patienten ist es dabei sinnvoll, wenn die einzelnen Leuchtsegmente in einem tragbaren Gehäuse bzw. in einem Handteil untergebracht sind, so dass die erfindungsgemäße Bestrahlungsvorrichtung beispielsweise auch zur Bestrahlung eines Bereichs der Mundhöhle eines Patienten eingesetzt werden kann.

Es werden nachfolgend unter Bezugnahme auf die beigefügte Zeichnung unterschiedliche Ausführungsbeispiele einer erfindungsgemäßen Bestrahlungsvorrichtung bzw. eines erfindungsgemäßen Bestrahlungssystems erläutert, welche beispielsweise zur medizinischen Photodynamischen Diagnose oder Therapie geeignet sind, wobei insbesondere Bestrahlungsvorrichtungen bzw. Bestrahlungssysteme vorgestellt werden, welche zur Bestrahlung von Körperregionen oder zur Ganzkörperbestrahlung eingesetzt werden können. Ebenso ist mit Hilfe der vorliegenden Erfindung die Bestrahlung von Hautoberflächen z.B. zur Behandlung von Akne ohne vorherige Gabe von Photosensibilatoren möglich, da die hohe Bestrahlungsstärke zur Behandlung von Akne ausreicht und die Akne auslösenden Bakterien selbst Protoporphyrin produzieren. Darüber hinaus ist die vorliegende Erfindung jedoch auch für kosmetische Anwendungen, beispielsweise zur Haarbleichung oder Haarentfernung, oder für industrielle Anwendungen, beispielsweise zur Einkopplung von Licht in einen Lichtwellenleiter (vorzugsweise mit Hilfe einer Sammellinse) oder zur Aushärtung (beispielsweise der Rundumbeschichtung oder Kodierung eines Kabels) geeignet. Ebenso kann die vorliegende Erfindung zur Rundumbestrahlung von transparenten Röhrchen oder Kapillaren mit darin befindlichen Flüssigkeiten, z.B. Blut, oder Zellkulturen etc. verwendet werden. Selbstverständlich ist die vorliegende Erfindung jedoch nicht auf die nachfolgend näher beschriebenen Anwendungsgebiete beschränkt, sondern kann allgemein überall dort zur Anwendung kommen, wo mit relativ einfachen Mitteln die Erzielung einer relativ hohen Bestrahlungsstärke mit einer insbesondere homogenen Bestrahlungsstärkeverteilung auf einer gewünschten Bestrahlungsfläche gewünscht ist.
Fig. 1 zeigt eine Querschnittsansicht einer Bestrahlungsvorrichtung gemäß einem ersten Ausführungsbeispiel der vorliegenden Erfindung,
Fig. 2 zeigt eine perspektivische Ansicht einer Bestrahlungsvorrichtung gemäß einem zweiten Ausführungsbeispiel der vorliegenden Erfindung,
Fig. 3 zeigt eine Querschnittsansicht einer Bestrahlungsvorrichtung gemäß einem dritten Ausführungsbeispiel der vorliegenden Erfindung,
Fig. 4 zeigt die Anordnung der einzelnen Leuchtsegmente des in Fig. 3 dargestellten Ausführungsbeispiels in Draufsicht,
Fig. 5 zeigt ein Bestrahlungssystem mit zwei Bestrahlungsvorrichtungen gemäß einem vierten Ausführungsbeispiel der vorliegenden Erfindung,
Fig. 6 zeigt eine Querschnittsansicht einer Bestrahlungsvorrichtung gemäß einem fünften Ausführungsbeispiel der vorliegenden Erfindung,
Fig. 7 zeigt eine Querschnittsansicht einer Bestrahlungsvorrichtung gemäß einem sechsten Ausführungsbeispiel der vorliegenden Erfindung,
Fig. 8 zeigt eine Querschnittsansicht einer Bestrahlungsvorrichtung gemäß einem siebten Ausführungsbeispiel der vorliegenden Erfindung,
Fig. 9 zeigt eine Querschnittsansicht einer Bestrahlungsvorrichtung gemäß einem achten Ausführungsbeispiel der vorliegenden Erfindung,
Fig. 10A und Fig. 10B zeigen Beispiele für eine regelmäßige Anordnung von Leuchtdioden in einem Leuchtsegment gemäß der vorliegenden Erfindung,
Fig. 11 zeigt Darstellungen einer Bestrahlungsvorrichtung gemäß einem neunten Ausführungsbeispiel der vorliegenden Erfindung.
Fig. 12 zeigt die Verwendung einer erfindungsgemäßen Bestrahlungsvorrichtung zur Einkopplung von Licht in einen Lichtwellenleiter,
Fig. 13A und Fig. 13B zeigen Beispiele für die Verwendung einer erfindungsgemäßen Bestrahlungsvorrichtung zur Rundumbestrahlung eines länglichen Mediums, um beispielsweise die Rundumbeschichtung oder Kodierung eines Kabels auszuhärten oder ein transparentes Röhrchen oder eine transparente Kapillare mit einem darin befindlichen biologischen Material (z.B. Blut oder eine Flüssigkeit mit Zellkulturen) zu bestrahlen,
Fig. 14 zeigt eine erfindungsgemäße Bestrahlungsvorrichtung, welche ähnlich zu der in Fig. 3 gezeigten Bestrahlungsvorrichtung aufgebaut ist, zur Ermöglichung einer Photodynamischen Diagnose (PDD),
Fig. 15A und Fig. 15B zeigen Darstellungen zur Erläuterung der Anordnung von Leuchtmitteln, beispielsweise Leuchtdioden, unterschiedlicher Wellenlänge auf einem Leuchtsegmentträger einer erfindungsgemäßen Bestrahlungsvorrichtung, und
Fig. 16 zeigt eine Darstellung, um die im Rahmen der beigefügten Figuren gewählte vereinfachte Darstellungsform für die Leuchtsegmentträger der erfindungsgemäßen Bestrahlungsvorrichtung zu verdeutlichen.

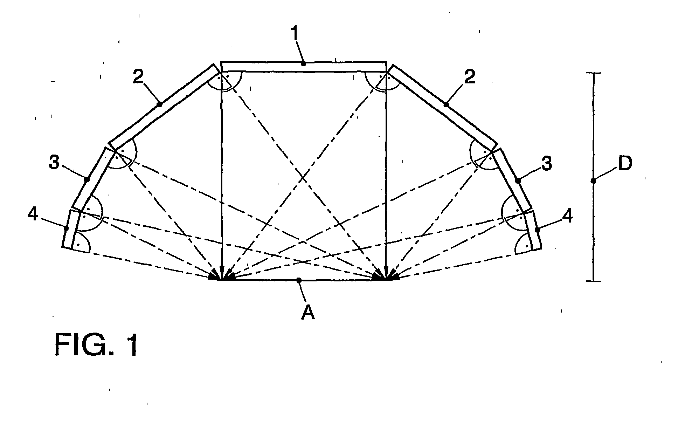

In Fig. 1 ist eine Querschnittsansicht einer erfindungsgemäßen Bestrahlungsvorrichtung dargestellt, wobei mehrere Leuchtsegmentträger mit entsprechenden Leuchtsegmenten 1-4 zueinander winkelig angeordnet sind. Jedes Leuchtsegment 1-4 ist dabei vorzugsweise durch eine regelmäßige Anordnung von geeigneten Leuchtmitteln, wie insbesondere Leuchtdioden oder Laserdioden, gebildet.

Wie aus Fig. 1 ersichtlich ist, erzeugen die einzelnen Leuchtsegmente 1-4 jeweils eine gerichtete Strahlung, wobei näherungsweise davon ausgegangen wird, dass die einzelnen Leuchtsegmente 1-4 wie in Fig. 1 dargestellt, in Bezug auf die Oberfläche des Leuchtsegmentes senkrecht abstrahlen. Die Leuchtsegmente 1-4 sind aneinandergrenzend bzw. benachbart zueinander derart angeordnet, dass bei Annäherung der Bestrahlungsvorrichtung an eine gewünschte Bestrahlungsfläche eine nahezu exakte bzw. im Wesentlichen vollständige Überlagerung der einzelnen Strahlungen auftritt. Auf diese Weise kann eine deutliche Erhöhung der Bestrahlungsstärke erzielt werden, obwohl als Leuchtmittel beispielsweise Leuchtdioden oder Laserdioden eingesetzt werden, welche im Vergleich zu hochenergetischen Lampen eine deutlich reduzierte Bestrahlungsstärke aufweisen. Der Abstand D zwischen der Bestrahlungsfläche A und der Bestrahlungsvorrichtung, bei dem die zuvor beschriebene Fokussierung der einzelnen Strahlungen auftritt und welcher gemäß Fig. 1 durch den Abstand zwischen der Bestrahlungsfläche A und dem unmittelbar gegenüberliegenden Leuchtsegment 1 definiert ist, hängt dabei im Wesentlichen von der winkeligen Anordnung der einzelnen Leuchtsegmente 1-4 zueinander ab.

Wie bereits erwähnt worden ist, weisen bei dem dargestellten Ausführungsbeispiel die einzelnen Leuchtsegmente 1-4 eine rechteckige Grundfläche auf, wobei die Grundfläche der einzelnen Leuchtsegmente 1-4 auf die Bestrahlungsfläche A abgebildet wird, so dass sich die einzelnen Strahlungen der Leuchtsegmente 1-4 auf der Bestrahlungsfläche A ebenfalls in einem Bereich mit einer rechteckigen Grundfläche überlappen. Bei der Wahl der Grundfläche der einzelnen Leuchtsegmente 1-4 ist zu beachten, dass die Grundfläche des Leuchtsegments 1, welches unmittelbar senkrecht gegenüberliegend zu der Bestrahlungsfläche A angeordnet ist, unverändert auf die Bestrahlungsfläche A abgebildet wird, während die winkelig zu der Bestrahlungsfläche A angeordneten Leuchtsegmente 2-4 mehr oder weniger stark verzerrt auf die Bestrahlungsfläche A abgebildet werden. Je spitzer der Winkel zwischen der Bestrahlungsfläche A und dem von den Leuchtsegmenten 2-4 ausgehenden Lot ist, desto geringer kann die Grundfläche des jeweiligen Leuchtsegments 2-4 gewählt werden, da die Verzerrung der Abbildung des jeweiligen Leuchtsegments zunimmt.

Mit Ausnahme der Breite können jedoch die Abmessungen der einzelnen Leuchtsegmente 1-4, d.h. die Höhe bzw. Dicke und die Tiefe, identisch gewählt sein.

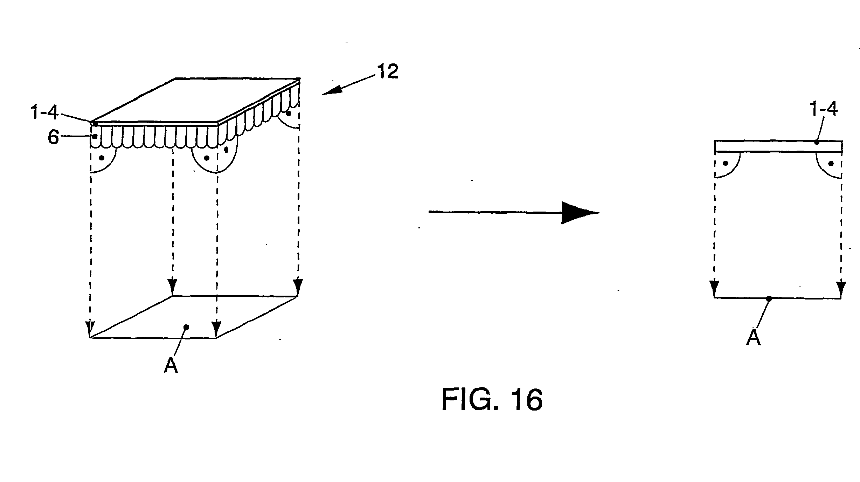

Um die in Fig. 1 sowie in den weiteren Figuren gewählte Darstellungsform der einzelnen Leuchtsegmentträger bzw. Leuchtsegmente 1-4 zu verdeutlichen, ist in Fig. 16 in perspektivischer Ansicht ein Beispiel für einen derartigen erfindungsgemäßen Leuchtsegmentträger 12 dargestellt. Wie aus der linken Darstellung von Fig. 16 ersichtlich ist, umfasst der Leuchtsegmentträger 12 eine Vielzahl von gleichmäßig auf seiner Oberfläche verteilten Leuchtmitteln 6, beispielsweise Laserdioden oder Leuchtdioden, welche eine gerichtete Strahlung derart erzeugen, dass die insgesamt von dem daraus resultierenden Leuchtsegment 1-4 erzeugte Strahlung im wesentlichen senkrecht auf die Bestrahlungsfläche A abgebildet wird. Auf diese Weise wird auf die Bestrahlungsfläche A eine Strahlung abgebildet, deren Grundfläche von der Grundfläche des jeweiligen Leuchtsegments 1-4, d.h. von der Grundfläche der Anordnung der einzelnen Leuchtmittel 6, abhängt. Bei dem in Fig. 16 dargestellten Beispiel ist die Bestrahlungsfläche A parallel zu dem Leuchtsegmentträger 12 bzw. dem entsprechenden Leuchtsegment 1-4 angeordnet, so dass die Form der Bestrahlungsfläche A der Form des jeweiligen Leuchtsegments 1-4 entspricht. Wie in der rechten Darstellung von Fig. 16 gezeigt ist, wird in den beigefügten Figuren in der Regel auf die separate Darstellung eines Leuchtsegmentträgers mit den einzelnen daran befindlichen Leuchtmitteln verzichtet, sondern es wird vereinfacht lediglich das der Anordnung der Leuchtmittel 6 auf dem Leuchtsegmentträger 12 entsprechende Leuchtsegment 1-4 dargestellt, wie es in der rechten Darstellung von Fig. 16 beispielsweise in einer Querschnittsansicht gezeigt ist.

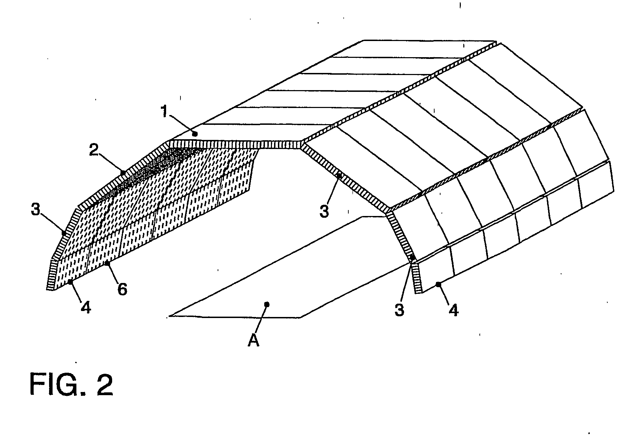

Fig. 2 zeigt ein Bestrahlungssystem, welches beispielsweise zur Ganzkörperbestrahlung eines Patienten zur medizinischen Photodynamischen Therapie (PDT) eingesetzt werden kann und auf dem in Fig. 1 dargestellten Aufbau basiert. Aus Fig. 2 ist insbesondere ersichtlich, dass mehrere Gruppen von Leuchtsegmenten 1-4 in Längsrichtung des Beleuchtungssystems aneinander angrenzend bzw. zueinander benachbart angeordnet sind, so dass insgesamt eine halbzylinderförmige Röhre gebildet wird, an deren Innenfläche die einzelnen Leuchtsegmente mit jeweils einer Vielzahl von Leuchtmitteln 6 angeordnet sind. Die von den einzelnen Leuchtsegmenten 1-4 ausgehende Strahlung ist somit in das Innere dieser halbzylinderförmigen Röhre gerichtet, so dass eine relativ große Bestrahlungsfläche A, deren Längsausdehnung der Länge des dargestellten Bestrahlungssystems entspricht, homogen und mit einer hohen Bestrahlungsstärke bestrahlt werden kann.

Aus Fig. 2 ist auch ersichtlich, dass die Tiefe der einzelnen Leuchtsegmente 1-4, d.h. die Abmessung in Längsrichtung der dargestellten halbzylinderförmigen Röhre, identisch ist, wobei die senkrecht zur Längsrichtung der halbzylinderförmigen Röhre verlaufenden Mittelachsen der Leuchtsegmente 1-4 zueinander ausgerichtet sind, so dass die Leuchtsegmente 1-4 einer Leuchtsegmentgruppe zueinander bündig verlaufen.

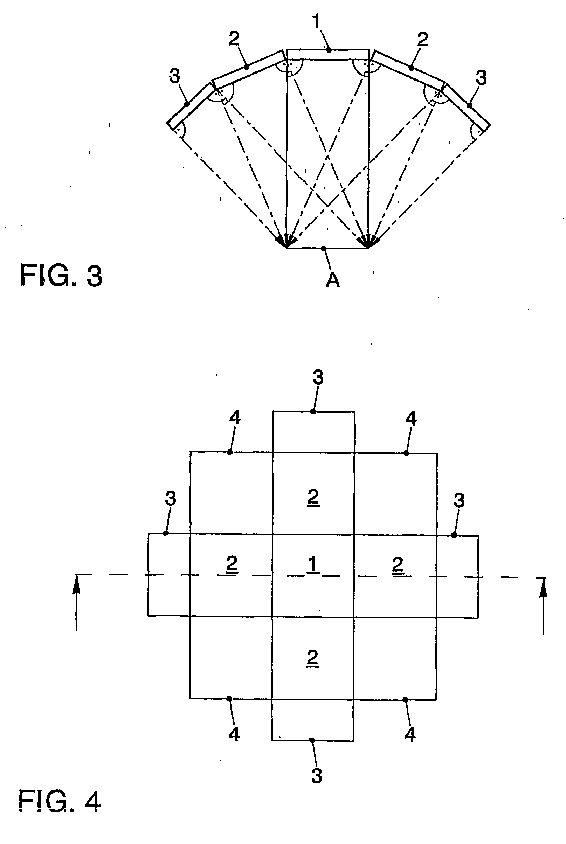

In Fig. 3 ist ein weiteres Ausführungsbeispiel einer erfindungsgemäßen Bestrahlungsvorrichtung in Querschnittansicht dargestellt, wobei im Gegensatz zu Fig. 1 lediglich fünf Leuchtsegmente 1-3 verwendet werden, deren Strahlungen sich vollständig, d.h. exakt, auf der Bestrahlungsfläche A überlappen. Dabei kann die in Fig. 3 dargestellte Bestrahlungsvorrichtung insbesondere Teil eines Bestrahlungssystems sein, dessen Leuchtsegmente in eine Ebene projiziert, d.h. in die Ebene des Leuchtsegments 1 aufgeklappt, die in Fig. 4 dargestellte Anordnung und Form aufweisen können, wobei die in Fig. 3 dargestellte Querschnittsansicht entlang einer in Fig. 4 gestrichelt dargestellten Schnittlinie in Pfeilrichtung genommen worden ist.

Wie aus Fig. 4 ersichtlich ist, sind die in Fig. 3 in Querschnittsansicht dargestellten Leuchtsegmente 1-3 in einer Reihe angeordnet, wobei.senkrecht zu dieser Reihe das Leuchtsegment 1 benachbart zu zwei weiteren Leuchtsegmenten 2 angeordnet ist, welche unter dem selben Winkel wie die beiden zuvor erwähnten Leuchtsegmente 2 bezüglich des Leuchtsegments 1 angeordnet sind. Ebenso sind zusätzlich zu den in Fig. 3 dargestellten Leuchtsegmenten 3 zwei weitere stirnseitige Leuchtsegmente 3 angeordnet, welche allesamt unter demselben Winkel bezüglich des Leuchtsegments 1 winkelig angeordnet sind. Zwischen zwei diagonal benachbart angeordneten Leuchtsegmenten 2 ist jeweils ein weiteres Leuchtsegment 4 vorgesehen, welches gegenüber den anderen Leuchtsegmenten 1-3 derart winkelig angeordnet ist, dass sich die von den einzelnen Leuchtsegmenten 1-4 abgegebenen Strahlungen auf der gewünschten Bestrahlungsfläche A im Wesentlichen vollständig überlappen, so dass auf der Bestrahlungsfläche A eine maximale Bestrahlungsintensität mit homogener Bestrahlungsstärkeverteilung auftritt.

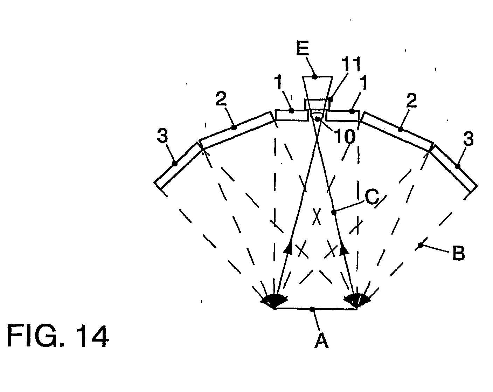

In Fig. 14 ist eine erfindungsgemäße Bestrahlungsvorrichtung zur medizinischen Photodynamischen Diagnose (PDD) dargestellt, wobei der Grundaufbau der in Fig. 14 gezeigten Bestrahlungsvorrichtung im wesentlichen dem Grundaufbau der in Fig. 3 gezeigten Bestrahlungsvorrichtung entspricht. Auch bei der in Fig. 14 gezeigten Bestrahlungsvorrichtung werden fünf Leuchtsegmente 1-3 verwendet, deren Strahlungen sich vollständig, d.h. exakt, auf der Bestrahlungsfläche A überlappen. Die Leuchtsegmente der Bestrahlungsvorrichtung können in die Ebene des Leuchtsegments 1 aufgeklappt die in Fig. 4 dargestellte Anordnung und Form aufweisen. Wird die Bestrahlungsvorrichtung derart positioniert, dass die Bestrahlungsfläche A auf einem gewünschten Körperabschnitt zu liegen kommt, wird durch die Strahlungen B der einzelnen Leuchtsegmente 1-3, welche sich auf der Bestrahlungsfläche A überlappen, in Abhängigkeit von einer dem Patienten verabreichten lichtempfindlichen Substanz, dem sogenannten Photosensibilisator, eine Fluoreszenzstrahlung C hervorgerufen, welche von der Bestrahlungsfläche A abgestrahlt wird. Bei einer PDD-Anwendung kann die Wellenlänge der von den einzelnen Leuchtsegmenten 1-3 erzeugten Strahlungen beispielsweise im Bereich zwischen 370nm und 430nm liegen. In dem mittleren Leuchtsegment 1 befindet sich eine Öffnung vorzugsweise mit einer Linse 10, mit deren Hilfe die emittierte Fluoreszenzstrahlung C gebündelt und auf eine Beobachtungsebene E abgebildet wird. Zudem ist ein optisches Filter 11 vorgesehen, welches aus der von der Linse 10 detektierten Strahlung die gewünschte Fluoreszenzstrahlung, beispielsweise eine Porphyrin-Fluoreszenzstrahlung im Wellenlängenbereich 600nm-750nm, ausfiltert, so dass externe Störeinflüsse (z.B. durch externe Strahlungsquellen) eliminiert werden können. Über die Beobachtungsebene E kann die somit angeregte und detektierte Fluoreszenzstrahlung beispielsweise mit dem Auge oder einem geeigneten optischen Auswertungssystem (z.B. einem Kameraobjektiv) beobachtet und ausgewertet werden, um somit neoplastische Veränderungen, wie beispielsweise Tumore, auf der beobachteten Hautoberfläche zu erkennen.

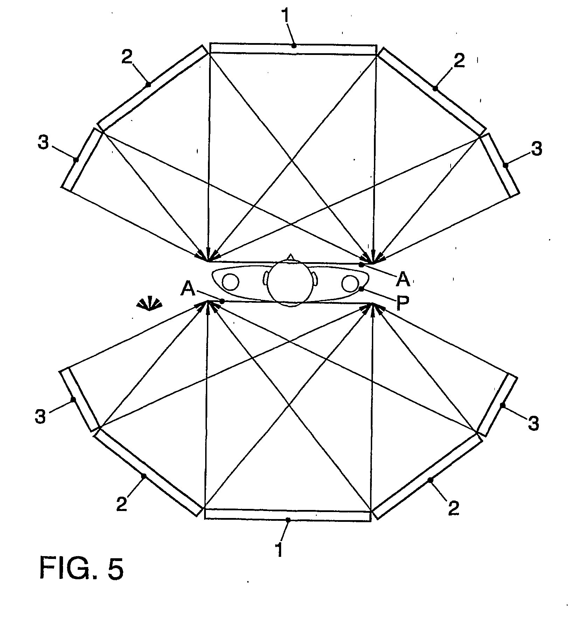

In Fig. 5 ist ein erfindungsgemäßes Bestrahlungssystem mit zwei Bestrahlungsvorrichtungen der in Fig. 1 dargestellten Art gezeigt, wobei die beiden Bestrahlungsvorrichtungen jedoch im Gegensatz zu Fig. 1 lediglich jeweils fünf Leuchtsegmente 1-3 umfassen. Wie aus Fig. 5 ersichtlich ist, ermöglicht das in Fig. 5 dargestellte Bestrahlungssystem, welches perspektivisch betrachtet analog zu Fig. 2 aufgebaut ist, eine Ganzkörperbestrahlung eines stehenden Patienten P, welcher im Bereich der Bestrahlungsflächen A der beiden Bestrahlungsvorrichtungen zu positionieren ist, wobei sich die Strahlungen der Leuchtsegmente 1-3 der oberen Bestrahlungsvorrichtung auf der oberen Bestrahlungsfläche A und die Strahlungen der Leuchtsegmente 1-3 der unteren Bestrahlungsvorrichtung auf der unteren Bestrahlungsfläche A vollständig überlappen. Selbstverständlich ist das in Fig. 5 gezeigte Bestrahlungssystem auch für die Ganzkörperbestrahlung eines liegenden Patienten P geeignet, falls der Patient P im Bereich der unteren Bestrahlungsfläche A auf einer strahlungsdurchlässigen Auflage, z.B. aus Quarz- oder Acrylglas, liegt.

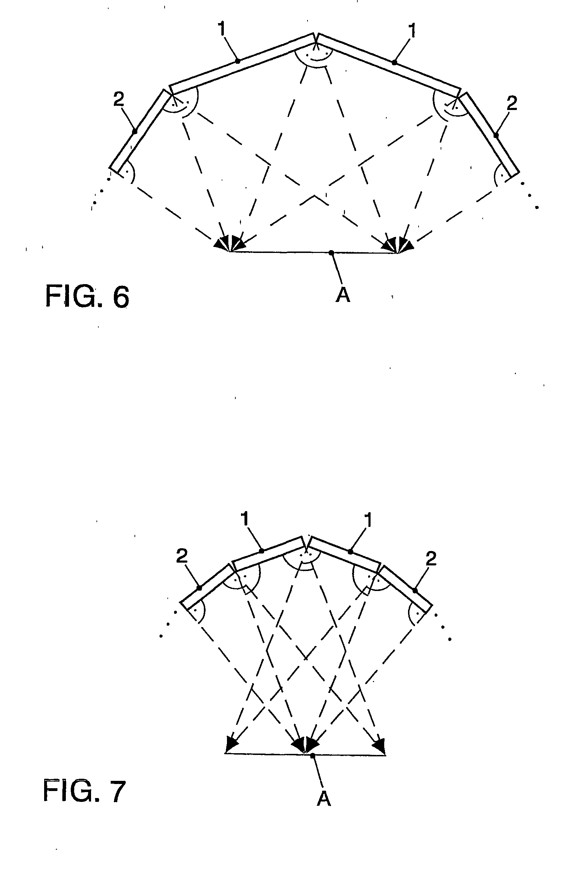

In Fig. 6 ist ein weiteres Ausführungsbeispiel einer erfindungsgemäßen Bestrahlungsvorrichtung dargestellt, wobei jeweils zwei Leuchtsegmente 1 und zwei Leuchtsegmente 2 vorgesehen sind, die derart winkelig zueinander angeordnet sind, dass sich die von den einzelnen Leuchtsegmenten 1, 2 erzeugte und im Wesentlichen senkrecht abgestrahlte Strahlung im Bereich der Bestrahlungsfläche A überlappt. Im Gegensatz zu den zuvor erläuterten Ausführungsbeispielen ist bei dem in Fig. 6 dargestellten Ausführungsbeispiel kein Leuchtsegment parallel zu der Bestrahlungsfläche A angeordnet, sondern sämtliche Leuchtsegmente 1, 2 sind in der dargestellten Querschnittsebene unter einem gewissen Winkel zu der Bestrahlungsfläche A angeordnet.

In Fig. 7 ist ein weiteres Ausführungsbeispiel dargestellt, welches ähnlich zu dem in Fig. 6 dargestellten Ausführungsbeispiel ausgestaltet ist, wobei jedoch die Anordnung der einzelnen Leuchtsegmente 1,2 derart gewählt ist, dass sich jeweils nur die von zwei benachbarten Leuchtsegmenten 1, 2 erzeugten Strahlungen überlappen, d.h. die von den beiden linken Leuchtsegmenten 1,2 erzeugten Strahlungen leuchten gemeinsam einen rechten Abschnitt der Bestrahlungsfläche A aus, während die von den beiden rechten Leuchtsegmenten 1, 2 erzeugten Strahlungen einen linken Abschnitt der Bestrahlungsfläche A ausleuchten, so dass jeder Bereich der Bestrahlungsfläche A von zwei Leuchtsegmenten 1, 2 bestrahlt wird. Auch bei diesem Ausführungsbeispiel ist somit eine homogene Ausleuchtung der Bestrahlungsfläche A mit relativ hoher Bestrahlungsintensität gegeben.

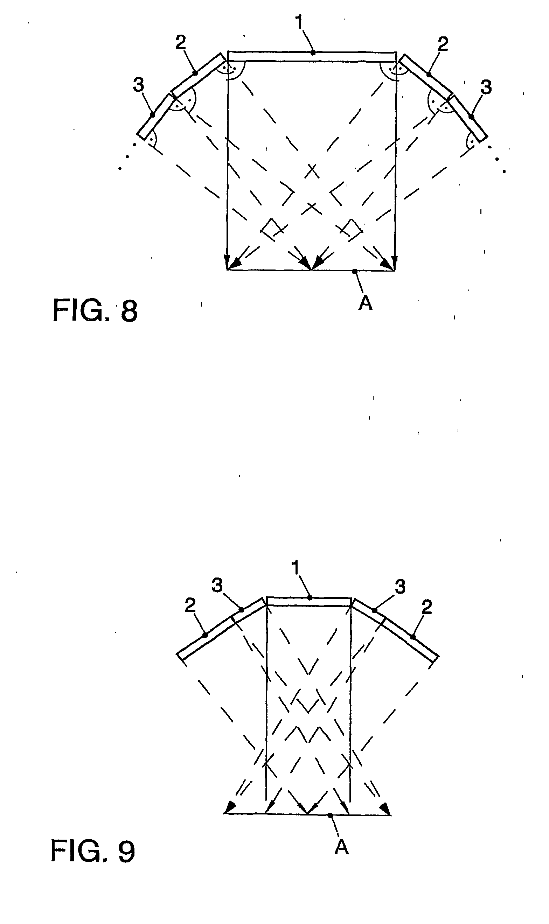

In Fig. 8 ist ein weiteres Ausführungsbeispiel dargestellt, wobei ein parallel zu der Bestrahlungsfläche A angeordnetes Leuchtsegment die gesamte Bestrahlungsfläche A ausleuchtet, während jeweils zwei winkelig zu dem Leuchtsegment 1 angeordnete Leuchtsegmente 2 und 3 lediglich die linke bzw. die rechte Hälfte der Bestrahlungsfläche A ausleuchten, so dass insgesamt jeder Bereich der Bestrahlungsfläche A durch drei Leuchtsegmente bestrahlt wird. Auch bei diesem Ausführungsbeispiel ist somit eine Bestrahlung der Bestrahlungsfläche A mit hoher Intensität und homogener Bestrahlungsstärkeverteilung gegeben.

Bei dem in Fig. 9 dargestellten Ausführungsbeispiel ist ebenfalls ein Leuchtsegment 1 parallel zur Bestrahlungsfläche A angeordnet, wobei zudem zwei winkelig zu dem Leuchtsegment 1 angeordnete Leuchtsegmente 2 jeweils die Hälfte des von dem Leuchtsegment 1 bestrahlten Abschnitts ausleuchten. Darüber hinaus sind zwei weitere winkelig angeordnete Leuchtsegmente 3 vorgesehen, welche diejenigen Abschnitte der Bestrahlungsfläche A ausleuchten, die nicht von dem Leuchtsegment 1 bestrahlt werden, so dass auch bei diesem Ausführungsbeispiel jeder Bereich der Bestrahlungsfläche A von jeweils zwei Leuchtsegmenten bestrahlt wird. Auch bei diesem Ausführungsbeispiel ist somit eine homogene Bestrahlung der Bestrahlungsfläche A mit hoher Bestrahlungsintensität gegeben, sofern die Bestrahlungsintensität der einzelnen Leuchtsegmente 1-3 entsprechend gewählt worden sind. Abhängig von der Wahl der Bestrahlungsintensität der einzelnen Leuchtsegmente 1-3 kann alternativ auch eine definierte Abschwächung oder Erhöhung der Bestrahlungsintensität in Teilbereichen der Bestrahlungsfläche A, z.B. in den von den Leuchtsegmenten 3 bestrahlten Randbereichen, erzielt werden.

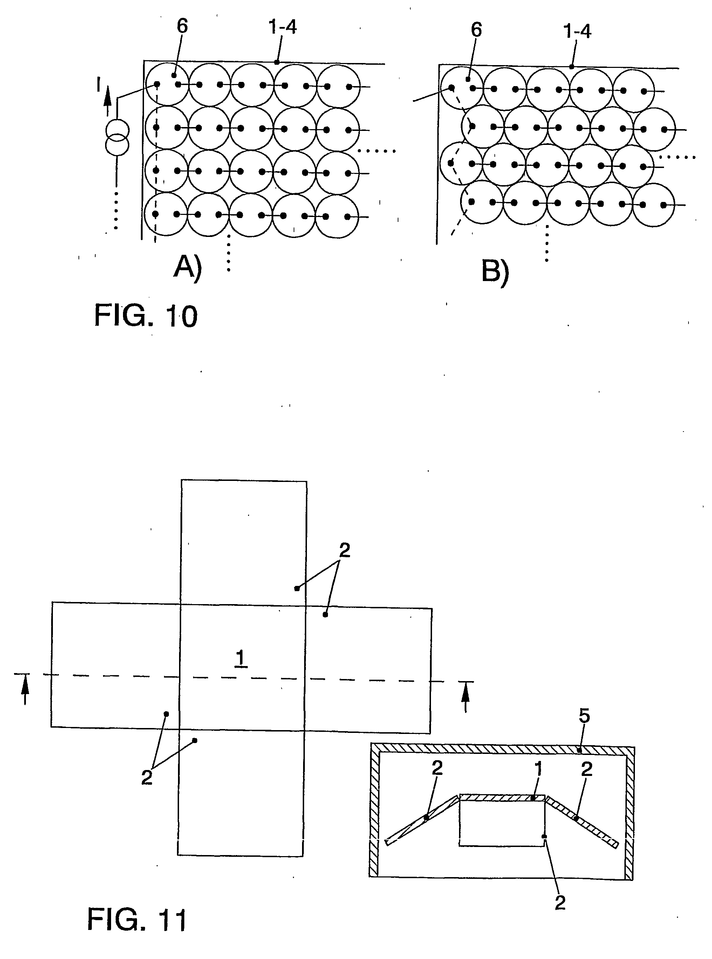

In Fig. 11 sind analog zu der in Fig. 4 gewählten Darstellungsform die einzelnen Leuchtsegmente 1, 2 eines weiteren Ausführungsbeispiels dargestellt, wobei zudem eine Querschnittsansicht der winkeligen Anordnung dieser Leuchtsegmente entlang einer gestrichelt dargestellten Schnittlinie gezeigt ist.

Wie aus Fig. 11 ersichtlich ist, ist ein zentrales Leuchtsegment 1 vorgesehen, zu dem sternförmig Leuchtsegmente 2 benachbart angeordnet sind. Die Leuchtsegmente 2 sind jeweils unter ein und demselben Winkel zu dem Leuchtsegment 1 nach unten abgewinkelt, wie aus der in Fig. 11 dargestellten Querschnittsansicht ersichtlich ist. Die Leuchtsegmente 1, 2 sind insbesondere derart angeordnet, dass ihre Grundfläche auf eine gemeinsame Bestrahlungsfläche abgebildet wird, welche sich bei dem dargestellten Ausführungsbeispiel parallel zu dem Leuchtsegment 1 unterhalb von demselben befindet. Darüber hinaus ist in Fig. 11 angedeutet, dass sich alle Leuchtsegmente 1, 2 in einem gemeinsamen Gehäuse 5 befinden können, welches an seiner Unterseite eine Austrittsöffnung für die von den einzelnen Leuchtsegmenten 1, 2 erzeugten Strahlungen aufweist. Dabei kann es sich bei dem Gehäuse 5 insbesondere um das Gehäuse eines Handteils handeln, so dass bei miniaturisierter Ausbildung der Leuchtsegmente 1, 2 beispielsweise die Bestrahlung eines Mundhöhlenabschnitts eines Patienten möglich ist. Bei Verwendung eines größeren (stationären) Gehäuses 5 ist z.B. die Bestrahlung des gesamten Gesichtsfelds eines Patienten möglich.

Wie bereits erwähnt worden ist, werden für die einzelnen Leuchtsegmente 1-4 der zuvor beschriebenen Ausführungsbeispiele bevorzugt jeweils eine Vielzahl von Leuchtmitteln verwendet, welche auf den einzelnen Leuchtsegmenten 1-4 gleichmäßig angeordnet sind. Entsprechende Ausführungsbeispiele sind in Fig. 10A und Fig. 10B dargestellt.

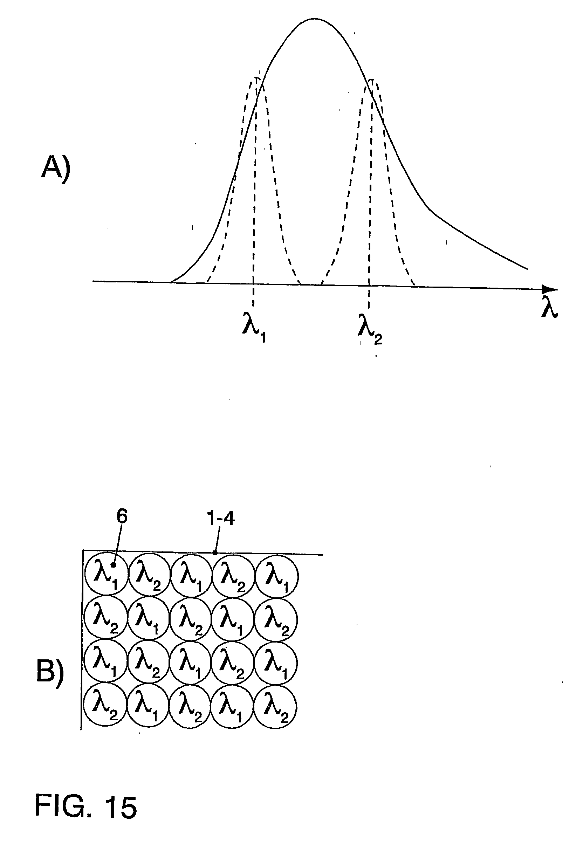

Als Leuchtmittel 6 kommen dabei bevorzugt insbesondere eng strahlende Leuchtdioden oder Laserdioden mit möglichst hoher Bestrahlungsintensität in Frage. Der Vorteil bei der Verwendung von Leuchtdioden oder Laserdioden ist, dass diese ein vorzugsweise selektiv schmalbandiges Emissionsspektrum erzeugen, welches für therapeutische/diagnostische Bestrahlungen mit möglichst hoher Selektivität in bestimmten Spektralbereichen, die vom jeweils verwendeten Photosensibilisator abhängig sind, verwendet werden kann. Sollte das gewünschte Behandlungsspektrum ein breitbandiges sein, kann dies durch Verwendung unterschiedlicher Leuchtmittel, welche z.B. im UV-Bereich, IR-Bereich oder im sichtbaren Lichtbereich emittieren, innerhalb eines Leuchtsegments erzielt werden (vgl. Fig. 15A zur Erzeugung eines optimalen PDD-Spektrums zur Fluoreszenzanregung oder z.B. bei einer PDT-Anwendung die Verwendung eines schmalbandigen Spektrums ohne Warmlichtanteile). Der Abstrahlwinkel der gewählten Leuchtdioden bzw. Laserdioden sollte möglichst schmal sein, wobei bei der Bestrahlung größerer Bestrahlungsflächen der Abstrahlwinkel weniger kritisch als bei kleineren Bestrahlungsflächen ist. Darüber hinaus ist der maximale Abstrahlwinkel der Leucht- bzw. Laserdioden vom Abstand D (vgl. Fig. 1) zu der Bestrahlungsfläche abhängig. Bei Versuchen hat sich gezeigt, dass ein maximaler Abstrahlwinkel von 30°, vorzugsweise 20°, eingehalten werden sollte, wobei jedoch idealerweise auch Leucht- bzw. Laserdioden mit einem Abstrahlwinkel von 6° oder sogar 3° zur Anwendung kommen können. Die Verwendung von eng strahlenden Leuchtdioden oder Laserdioden ist vorteilhaft, da für die Realisierung der vorliegenden Erfindung die Erzeugung von gerichteten Strahlungen durch die einzelnen Leuchtsegmente erforderlich ist, da nur in diesem Fall eine exakte Überlappung der einzelnen Strahlungen auf der gewünschten Bestrahlungsfläche und daraus resultierend eine homogene Bestrahlung der Bestrahlungsfläche mit hoher Bestrahlungsintensität gewährleistet ist.

Wie in Fig. 10A gezeigt ist, können für die Leuchtsegmente 1-4 beispielsweise Leuchtdioden 6 verwendet werden, welche matrixartig in Reihen und Spalten gleichmäßig angeordnet sind. Eine gleichmäßige Anordnung der Leuchtdioden auf den einzelnen Leuchtsegmenten 1-4 ist vorteilhaft, um eine homogene Bestrahlung der gewünschten Bestrahlungsfläche zu erzielen. Anstelle einer matrixartigen Anordnung der einzelnen Leuchtdioden können die Leuchtdioden 6 auch gemäß Fig. 10B in Reihen angeordnet sein, wobei die Reihen abwechselnd zueinander versetzt angeordnet sind.

Die einzelnen Leuchtdioden (oder Leuchtmittel) 6 ein und desselben Leuchtsegments 1-4 emittieren vorzugsweise Licht desselben Wellenlängenspektrums.

Ebenso ist jedoch auch denkbar, dass auf den einzelnen Leuchtsegmenten 1-4 bzw. den entsprechenden Leiterplatten mehrere Gruppen von Leuchtdioden 6 vorgesehen sind, wobei die Leuchtdioden jeweils innerhalb der einzelnen Gruppen jeweils gleichmäßig über das entsprechende Leuchtsegment verteilt sind und die Leuchtdioden 6 der einzelnen Gruppen Licht unterschiedlicher Wellenlänge emittieren, so dass durch selektive Ansteuerung bzw. Aktivierung der Leuchtdioden jeweils einer Gruppe ein und dasselbe Leuchtsegment zur Emission von Licht unterschiedlicher Wellenlängen verwendet werden kann. Ein Beispiel für eine derartige Anordnung von Leuchtdioden 6, welche in zwei Gruppen unterschiedlicher Wellenlänge bzw. unterschiedlicher Wellenlängenspektren unterteilt sind, ist in Fig. 15B dargestellt. Wie aus Fig. 15B ersichtlich ist, erzeugen die Leuchtdioden der einen Leuchtdiodengruppe eine Strahlung im Bereich einer Hauptwellenlänge λ₁, während die Leuchtdioden der anderen Leuchtdiodengruppe eine Strahlung im Bereich einer Hauptwellenlänge λ₂ erzeugen. Die Leuchtdioden einer Leuchtdiodengruppe sind jeweils gleichmäßig über das entsprechende Leuchtsegment 1-4 verteilt, so dass sich insgesamt die in Fig. 15B gezeigte gleichmäßige Anordnung von Leuchtdioden 6, die Licht unterschiedlicher Wellenlängenspektren emittieren, ergibt, d.h. es ist jeweils abwechselnd eine Leuchtdiode 6 mit der Hauptwellenlänge λ₁ und eine Leuchtdiode 6 mit der Hauptwellenlänge λ₂ auf dem Leuchtsegment 1-4 angeordnet.

Wie in Fig. 15A gezeigt ist, wird abhängig von der Aktivierung entweder der Leuchtdioden der ersten Leuchtdiodengruppe oder der Leuchtdioden der zweiten Leuchtdiodengruppe eine Strahlung mit der Hauptwellenlänge λ₁ oder der Hauptwellenlänge λ₂ erzeugt. Bei gleichzeitiger Aktivierung sämtlicher Leuchtdioden beider Leuchtdiodengruppen ergibt sich ein dem Wunschspektrum möglichst nahe kommendes und in Fig. 15A gezeigtes Gesamtspektrum der von dem jeweiligen Leuchtsegment 1-4 abgestrahlten Strahlung. Diese Anordnung kann z.B. dann gewählt werden, wenn für das Wunschspektrum kein Leuchtmittel existiert und daher durch Verwendung mehrerer unterschiedlicher Leuchtmittel ein dem Wunschspektrum möglichst nahe kommendes Spektrum erzielt werden muss.

Die Verwendung von Leuchtdiodengruppen unterschiedlicher Wellenlängenspektren auf ein und demselben Leuchtsegment 1-4 ist insbesondere dann vorteilhaft, wenn die Bestrahlungsvorrichtung beispielsweise für medizinische photodynamische Therapieanwendungen mit verschiedenen Photosensibilisatoren eingesetzt werden soll. Da diese Photosensibilisatoren üblicherweise bei unterschiedlichen Wellenlängen reagieren, kann auf diese spektralen Unterschiede durch entsprechende Aktivierung der jeweiligen Leuchtdiodengruppe eingegangen werden. Gleiches gilt für medizinische photodynamische Diagnoseanwendungen, wobei auf diese Weise je nach Art des fluoreszierenden Stoffes unterschiedliche Anregungsspektren verwendet werden können.

Wie in Fig. 10A angedeutet ist, werden die einzelnen Leuchtdioden 6 vorzugsweise mit Konstantstrom I einer Konstantstromquelle betrieben, wobei mehrere Leuchtdioden 6, beispielsweise 16 Leuchtdioden, jeweils in Reihe geschaltet sein können. Die Ansteuerung der einzelnen Leuchtdioden 6 mit Konstantstrom ist vorteilhaft, da auf diese Weise eine gleichmäßigere Helligkeit der Leuchtdioden 6 ein und desselben Leuchtsegments 1-4 erzielt werden kann. Darüber hinaus kann bei Verwendung von Konstantstrom die Helligkeit der einzelnen Leuchtdioden 6 leichter geregelt werden. Für die einzelnen Leuchtdiodenreihen kann eine gemeinsame Konstantstromquelle (Parallelbetrieb) vorgesehen sein. Ebenso ist jedoch auch möglich, für jede Leuchtdiodenreihe oder andere Gruppierungen von Leuchtdioden separate Konstantstromquellen zu verwenden.

Anhand der zuvor beschriebenen Ausführungsbeispiele wurde insbesondere der bevorzugte Anwendungsbereich einer medizinischen photodynamischen Diagnose oder Therapie beschrieben. Zur Durchführung der photodynamischen Therapie können die in der jeweiligen Bestrahlungsvorrichtung eingesetzten Leuchtsegmente insbesondere derart ausgestaltet sein, dass sie Strahlung im roten Bereich, insbesondere im Bereich um 635 nm emittieren. Zur photodynamischen Diagnose kommt hingegen in der Regel eine Anregungsstrahlung im UV- oder UV-nahen Bereich in Frage, wobei hier die Wellenlänge insbesondere zwischen 370 nm und 430 nm liegen sollte. Bei Verwendung des in Fig. 15A und Fig. 15B dargestellten Prinzips von Leuchtmitteln bzw. Leuchtdioden unterschiedlicher Wellenlängenspektren kann somit z.B. λ₁=370 nm und λ₂=430 nm gewählt werden.

Neben den zuvor genannten Anwendungsgebieten sind jedoch für die vorliegende Erfindung eine Reihe weiterer Anwendungsfelder denkbar, die beispielsweise im kosmetischen oder industriellen Bereich liegen können. So kann die erfindungsgemäße Bestrahlungsvorrichtung bzw. das erfindungsgemäße Bestrahlungssystem beispielsweise auch für kosmetische Zwecke zur Haarbleichung oder Haarentfernung eingesetzt werden, wobei zu diesem Zweck beispielsweise Wellenlängen im sichtbaren Bereich (VIS-Bereich), insbesondere im Bereich um 500 nm, zur Anwendung kommen können. Ebenso können Wellenlängen >800 nm (IR-Dioden) oder <380 nm (UV-Dioden) verwendet werden.

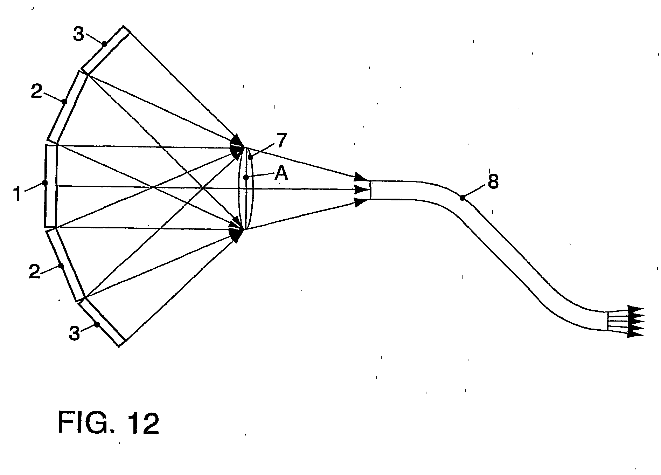

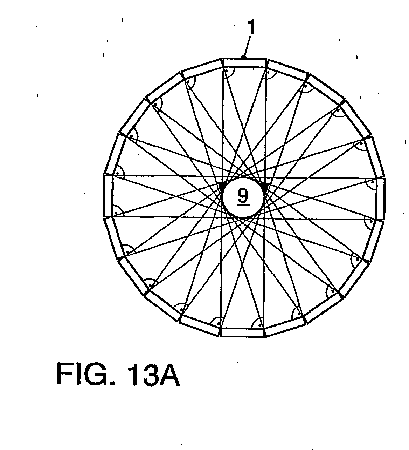

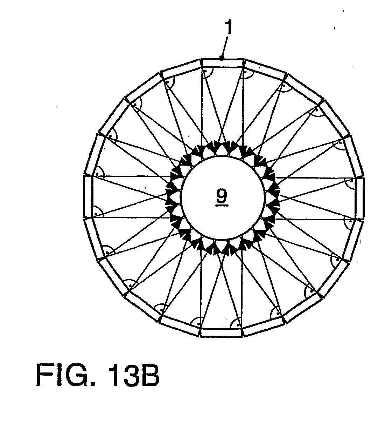

Weitere bevorzugte Anwendungsbereiche sollen nachfolgend unter Bezugnahme auf Fig. 12 und Fig. 13A sowie Fig. 13B erläutert werden.

In Fig. 12 ist eine Bestrahlungsvorrichtung der in Fig. 3 dargestellten Art mit Leuchtsegmenten 1-3 gezeigt, welche derart winkelig angeordnet sind, dass die von den einzelnen Leuchtsegmenten 1-3 senkrecht abgestrahlten Strahlungen auf einer Bestrahlungsfläche A gebündelt bzw. fokussiert werden, d.h. die von den einzelnen Leuchtsegmenten 1-3 abgestrahlten Strahlungen überlappen sich vollständig auf dieser Bestrahlungsfläche A. Die Anordnung ist derart gewählt, dass die Bestrahlungsfläche A auf bzw. in einer Sammellinse 7 liegt, durch welche die eintreffende Strahlung in einen Lichtwellenleiter 8 eingekoppelt wird. Der Vorteil der in Fig. 12 gezeigten Anordnung besteht somit darin, dass durch die Überlappung der von den einzelnen Leuchtsegmenten 1-3 erzeugten Strahlungen Licht mit hoher Bestrahlungsintensität auf einfache Art und Weise für die Einkopplung in einen Lichtwellenleiter 8 erzeugt werden kann. Bei Fokussierung der Strahlungen der Leuchtsegmente 1-3 auf die Lichteintrittsfläche des Lichtwellenleiters 8 kann die Sammellinse 7 auch entfallen.

Bei dem in Fig. 13A dargestellten Anwendungsfall ist eine Vielzahl von identisch aufgebauten Leuchtsegmenten 1 derart angeordnet, dass sie im Querschnitt eine kreisrunde Form bilden. Jedes Leuchtsegment 1 erzeugt eine im Wesentlichen senkrecht abgestrahlte Strahlung gleichen Durchmessers. Auf diese Weise wird ein Bereich um den Mittelpunkt der in Fig. 13A gezeigten Querschnittsform gleichmäßig von allen Leuchtsegmenten 1 bestrahlt, so dass in diesem Bereich eine Bestrahlung mit maximaler und gleichmäßig verteilter Bestrahlungsintensität vorhanden ist.

Die in Fig. 13A gezeigte Anordnung kann beispielsweise verwendet werden, um ein in diesem Überlappungsbereich der Strahlungen der einzelnen Leuchtsegmente 1 angeordneten Körper 9 bzw. dessen Oberfläche auszuhärten. Weisen die einzelnen Leuchtsegmente 1 eine entsprechende Länge auf oder sind mehrere der in Fig. 13A gezeigten Bestrahlungsvorrichtungen in Längsrichtung zueinander benachbart angeordnet, so dass durch die Leuchtsegmente 1 eine zylinderförmige Röhre gebildet wird, kann mit dieser Anordnung beispielsweise die Beschichtung oder Kodierung eines entlang der Mittelachse dieser Röhre angeordneten Kabels 9 ausgehärtet werden. Bei dem von den einzelnen Leuchtsegmenten 1 bestrahlten Körper 9, der im Überlappungsbereich der Strahlungen der einzelnen Leuchtsegmente 1 angeordnet ist, kann es sich beispielsweise auch um einen in Längsrichtung der Bestrahlungsvorrichtung verlaufenden Körper aus einem lichtdurchlässigen bzw. transparenten Material, beispielsweise ein Röhrchen oder eine Kapillare, handeln, so dass darin befindliche Flüssigkeiten (z.B. Blut) oder in Flüssigkeiten transportierte Zellen oder Stoffe mit dem Licht angeregt, beleuchtet bzw. dargestellt (markiert) werden können. Auf diese Weise ist beispielsweise auch die Durchführung einer Photodynamischen Diagnose oder Photodynamischen Therapie von einzelnen Blutzellen oder anderen Zellkulturen, die in Flüssigkeiten transportiert werden, möglich.

Bei dem in Fig. 13A gezeigten Beispiel entspricht der Durchmesser der von einzelnen Leuchtsegmenten 1 erzeugten Strahlungen im wesentlichen dem Querschnittsdurchmesser des im Überlappungsbereich der einzelnen Strahlungen angeordneten Körpers 9. Selbstverständlich ist jedoch auch denkbar, dass die Anordnung der Leuchtsegmente 1 sowie die Durchmesser der von den einzelnen Leuchtsegmenten 1 erzeugten Strahlungen derart gewählt werden, dass die Durchmesser der Strahlungen jeweils kleiner als der Durchmesser des rundum zu bestrahlenden Körpers 9 ist und sich die einzelnen Strahlungen auf der Oberfläche des zu bestrahlenden Körpers 9 gleichmäßig überlappen, wie es in Fig. 13B dargestellt ist. Insbesondere ist die Anordnung derart, dass jeder Oberflächenpunkt des Körpers 9 von mindestens zwei, bei dem dargestellten Ausführungsbeispiel sogar von drei Leuchtsegmenten 1 bestrahlt wird. Ansonsten gelten für die in Fig. 13B gezeigte Bestrahlungsvorrichtung analog die Erläuterungen zu Fig. 13A.

## Patentansprüche

1. Bestrahlungsvorrichtung,
mit mindestens zwei Leuchtsegmentträgern (12), wobei jeder Leuchtsegmentträger ein flächiges Leuchtsegment (1-4) mit einer Vielzahl von gleichmäßig angeordneten Leuchtmitteln (6) umfasst, wobei die Leuchtsegmentträger (12) winkelig zueinander angeordnet sind,
**dadurch gekennzeichnet,**
**dass** der Abstrahlwinkel der einzelnen Leuchtmittel (6) maximal 30° beträgt, so dass von jedem Leuchtsegment (1-4) eine gerichtete und in Bezug auf die Oberfläche des Leuchtsegmentes im Wesentlichen senkrecht abgestrahlte Strahlung erzeugt wird,
und
**dass** die Leuchtsegmentträger (12) derart winkelig zueinander angeordnet sind, dass sich die von den einzelnen Leuchtsegmenten (1-4) im Wesentlichen senkrecht abgestrahlten Strahlungen bei Anordnung der Bestrahlungsvorrichtung mit einem bestimmten Abstand (D) zu einer Bestrahlungsfläche (A) auf dieser Bestrahlungsfläche (A) im Wesentlichen vollständig überlappen, derart daß jeder Abschnitt der Bestrahlungsfläche durch die überlappende Strahlung von mindestens zwei Leuchtsegmenten bestrahlt wird.

2. Bestrahlungsvorrichtung nach Anspruch 1,
**dadurch gekennzeichnet,**
**dass** die Leuchtmittel (6) auf den einzelnen Leuchtsegmenten (1-4) gleichmäßig in Spalten und Reihen angeordnet sind.

3. Bestrahlungsvorrichtung nach Anspruch 1,
**dadurch gekennzeichnet,**
**dass** die Leuchtmittel (6) in den einzelnen Leuchtsegmenten (1-4) in abwechselnd zueinander versetzten Reihen angeordnet sind.

4. Bestrahlungsvorrichtung nach einem der vorhergehenden Ansprüche,
**dadurch gekennzeichnet,**
**dass** eine Energieversorgungseinrichtung zur Versorgung der einzelnen Leuchtmittel (6) der Leuchtsegmente (1-4) mit Konstantstrom vorgesehen ist.

5. Bestrahlungsvorrichtung nach Anspruch 4 und Anspruch 2 oder 3,
**dadurch gekennzeichnet,**
**dass** die jeweils in einer Reihe angeordneten Leuchtmittel (6) in Serie geschaltet und mit der Energieversorgungseinrichtung verbunden sind.

6. Bestrahlungsvorrichtung nach einem der vorhergehenden Ansprüche,
**dadurch gekennzeichnet,**
**dass** die Leuchtmittel (6) Leuchtdioden sind..

7. Bestrahlungsvorrichtung nach einem der Ansprüche 2-6,
**dadurch gekennzeichnet,**
**dass** die Leuchtmittel (6) Laserdioden sind.

8. Bestrahlungsvorrichtung nach einem der vorhergehenden Ansprüche,
**dadurch gekennzeichnet,**
**dass** die Leuchtsegmente (1-4) jeweils mehrere Gruppen von gleichmäßig angeordneten Leuchtmitteln (6) umfassen, wobei die Leuchtmittel (6) innerhalb einer Gruppe Strahlungen in einem im wesentlichen gleichen Wellenlängenbereich und die einzelnen Gruppen von Leuchtmitteln (6) Strahlungen in unterschiedlichen Wellenlängenbereichen erzeugen.

9. Bestrahlungsvorrichtung nach einem der vorhergehenden Ansprüche,
**dadurch gekennzeichnet,**
**dass** die einzelnen Leuchtsegmente (1-4) eine gerichtete Strahlung im ultravioletten Wellenlängenbereich erzeugen.

10. Bestrahlungsvorrichtung nach einem der Ansprüche,
**dadurch gekennzeichnet,**
**dass** die einzelnen Leuchtsegmente (1-4) eine gerichtete Strahlung im infraroten Wellenlängenbereich erzeugen.

11. Bestrahlungsvorrichtung nach einem der vorhergehenden Ansprüche,
**dadurch gekennzeichnet,**
**dass** die einzelnen Leuchtsegmente (1-4) eine gerichtete Strahlung im sichtbaren Wellenlängenbereich erzeugen.

12. Bestrahlungsvorrichtung nach einem der vorhergehenden Ansprüche,
**dadurch gekennzeichnet,**
**dass** die Grundfläche der einzelnen Leuchtsegmente (1-4) derart gewählt ist, dass sich die von den einzelnen Leuchtsegmenten (1-4) erzeugten Strahlungen im Wesentlichen vollständig auf einer rechteckigen Bestrahlungsfläche (A) überlappen.

13. Bestrahlungsvorrichtung nach einem der Ansprüche 1-11,
**dadurch gekennzeichnet,**
**dass** die Grundfläche der einzelnen Leuchtsegmente (1-4), derart gewählt ist, dass sich die von den einzelnen Leuchtsegmenten (1-4) erzeugten Strahlungen auf einer mehreckigen Bestrahlungsfläche (A) im Wesentlichen vollständig überlappen.

14. Bestrahlungsvorrichtung nach einem der Ansprüche 1-11,
**dadurch gekennzeichnet,**
**dass** die Grundfläche der einzelnen Leuchtsegmente (1-4) derart gewählt ist, dass sich die von den einzelnen Leuchtsegmenten (1-4) erzeugten Strahlungen auf einer runden Bestrahlungsfläche (A) im Wesentlichen vollständig überlappen.

15. Bestrahlungsvorrichtung nach einem der vorhergehenden Ansprüche,
**dadurch gekennzeichnet,**
**dass** die einzelnen Leuchtsegmentträger mit den entsprechenden Leuchtsegmenten (1-4) zueinander winkelig und zueinander benachbart angeordnet sind.

16. Bestrahlungsvorrichtung nach Anspruch 15,
**dadurch gekennzeichnet,**
**dass** die Leuchtsegmentträger mit den entsprechenden Leuchtsegmenten (1-4) derart zueinander benachbart angeordnet sind, dass durch die einzelnen Leuchtsegmente (1-4) eine im Wesentlichen ununterbrochene Abstrahlfläche gebildet ist.

17. Bestrahlungsvorrichtung nach einem der vorhergehenden Ansprüche,
**dadurch gekennzeichnet,**
**dass** die Leuchtsegmentträger mit den Leuchtsegmenten (1-4) in einem tragbaren Gehäuse (5) untergebracht sind.

18. Bestrahlungsvorrichtung nach einem der vorhergehenden Ansprüche,
**dadurch gekennzeichnet,**
**dass** die einzelnen Leuchtsegmente (1-4) derart zueinander ausgerichtet angeordnet sind, dass Mittelachsen der einzelnen Leuchtsegmente (1-4) in einer gemeinsamen Ebene verlaufen.

19. Bestrahlungsvorrichtung nach einem der vorhergehenden Ansprüche,
**dadurch gekennzeichnet,**
**dass** die Bestrahlungsvorrichtung mindestens drei zueinander winkelig angeordnete Leuchtsegmente (1-4) umfasst.

20. Bestrahlungsvorrichtung nach einem der vorhergehenden Ansprüche,
**dadurch gekennzeichnet,**
**dass** die Bestrahlungsvorrichtung eine erste Gruppe von zueinander winkelig angeordneten Leuchtsegmenten (1-3) und eine zweite Gruppe von zueinander winkelig angeordneten Leuchtsegmenten (1-3) umfasst, wobei die beiden Gruppen von Leuchtsegmenten (1-3) derart kreuzförmig angeordnet sind, dass ein bestimmtes Leuchtsegment (1) sowohl Bestandteil der ersten Gruppe als auch Bestandteil der zweiten Gruppe ist.

21. Bestrahlungsvorrichtung nach einem der vorhergehenden Ansprüche,
**dadurch gekennzeichnet,**
**dass** die Bestrahlungsvorrichtung mindestens vier Leuchtsegmente (1-4) umfasst.

22. Bestrahlungsvorrichtung nach einem der vorhergehenden Ansprüche,
**dadurch gekennzeichnet,**
**dass** die Bestrahlungsvorrichtung mindestens fünf winkelig zueinander angeordnete Leuchtsegmente (1-4) umfasst.

23. Bestrahlungsvorrichtung nach einem der vorhergehenden Ansprüche,
**dadurch gekennzeichnet,**
**dass** die Bestrahlungsvorrichtung mindestens sieben winkelig zueinander angeordnete Leuchtsegmente (1-4) umfasst.

24. Bestrahlungsvorrichtung nach einem der vorhergehenden Ansprüche,
**dadurch gekennzeichnet,**
**dass** die Bestrahlungsvorrichtung eine Detektoreinrichtung (10, 11) zum Erfassen von an der Bestrahlungsfläche (A) in Folge der Bestrahlung durch die Strahlungen der Leuchtsegmente (1-4) angeregte Fluoreszenzstrahlung (C) umfasst.

25. Bestrahlungsvorrichtung nach Anspruch 24,
**dadurch gekennzeichnet,**
**dass** die Detektoreinrichtung ein optisches Filter (11) mit einem der angeregten Fluoreszenzstrahlung (C) entsprechenden Durchlassbereich umfasst.

26. Bestrahlungsvorrichtung nach Anspruch 24 oder 25,
**dadurch gekennzeichnet,**
**dass** die Bestrahlungsvorrichtung Bestandteil eines medizinischen Photodynamischen Diagnosesystems ist.

27. Bestrahlungsvorrichtung nach einem der vorhergehenden Ansprüche,
**dadurch gekennzeichnet,**
**dass** die einzelnen Leuchtsegmente im Querschnitt im Wesentlichen halbkreisförmig angeordnet sind.

28. Bestrahlungsvorrichtung nach Anspruch 27,
**dadurch gekennzeichnet,**
**dass** die einzelnen Leuchtsegmente (1-4) im Wesentlichen halbzylinderförmig angeordnet sind.

29. Bestrahlungsvorrichtung nach Anspruch 28,
**dadurch gekennzeichnet,**
**dass** mehrere Gruppen von winkelig zueinander angeordneten Leuchtsegmenten (1-4) im Wesentlichen zueinander parallel angeordnet sind, so dass Leuchtsegmente (1-4) der einzelnen Gruppen im Wesentlichen halbzylinderförmig mit in das Innere des Halbzylinders gerichteter Strahlung angeordnet sind.

30. Bestrahlungsvorrichtung nach Anspruch 29,
**dadurch gekennzeichnet,**
**dass** die einzelnen, Leuchtsegmentgruppen unmittelbar aneinander angrenzen.

31. Bestrahlungssystem,
mit zwei einander gegenüberliegenden Bestrahlungsvorrichtungen nach einem der Ansprüche 28-30, wobei die Bestrahlungsfläche (A) der Bestrahlungsvorrichtungen zwischen den beiden Bestrahlungsvorrichtungen angeordnet ist.

32. Bestrahlungssystem nach Anspruch 31,
**dadurch gekennzeichnet,**
**dass** das Bestrahlungssystem zur Ganzkörperbestrahlung eines im Bereich der Bestrahlungsfläche (A) zu positionierenden Lebewesens (P) ausgestaltet ist.

33. Bestrahlungssystem mit einer Bestrahlungsvorrichtung nach einem der Ansprüche 1-30 zur Bestrahlung eines Körperabschnitts eines Lebewesens.

34. Bestrahlungssystem nach Anspruch 33,
**dadurch gekennzeichnet,**
**dass** die Bestrahlungsvorrichtung in einem mobilen Gehäuse (5) zur Positionierung der Bestrahlungsvorrichtung über dem gewünschten Körperabschnitt untergebracht ist.

35. Bestrahlungssystem nach einem der Ansprüche 31-34,
**dadurch gekennzeichnet,**
**dass** das Bestrahlungssystem Bestandteil eines medizinischen Photodynamischen Therapiesystems ist.

36. Bestrahlungssystem mit einer Bestrahlungsvorrichtung nach einem der Ansprüche 1-30 zur kosmetischen Behandlung eines Lebewesens.

37. Bestrahlungssystem,
mit einer Bestrahlungsvorrichtung nach einem der Ansprüche 1-30 zur Einkopplung der auf die Bestrahlungsfläche (A) treffenden überlappenden Strahlung in einen Lichtwellenleiter (8).

38. Bestrahlungssystem mit einer Bestrahlungsvorrichtung nach einem der Ansprüche 1-26,
wobei die Bestrahlungsvorrichtung eine Vielzahl von Leuchtsegmenten (1) umfasst, welche derart winkelig zueinander angeordnet sind, dass sie eine im Wesentlichen kreisförmige Querschnittsform bilden, so dass sich die von den einzelnen Leuchtsegmenten (1) erzeugten Strahlungen in einem mittleren Bereich dieser kreisförmigen Querschnittsform überlappen.

39. Bestrahlungssystem nach Anspruch 38,
**dadurch gekennzeichnet,**
**dass** die von den einzelnen Leuchtsegmenten (1) der Bestrahlungsvorrichtung erzeugten Strahlungen einen Durchmesser aufweisen, welcher im wesentlichen dem Durchmesser des mittleren Bereichs, in dem sich diese Strahlungen überlappen, entspricht.

40. Bestrahtungssystem nach Anspruch 38,
**dadurch gekennzeichnet,**
**dass** die von den einzelnen Leuchtsegmenten (1) der Bestrahlungsvorrichtung erzeugten Strahlungen einen Durchmesser aufweisen, welcher kleiner als der Durchmesser des mittleren Bereichs, in dem sich die Strahlungen überlappen, ist, so dass jeder Abschnitt dieses mittleren Bereichs gleichmäßig durch die Strahlungen von mindestens zwei Leuchtsegmenten (1) bestrahlt wird.

41. Bestrahlungssystem nach Anspruch 40,
**dadurch gekennzeichnet,**
**dass** jeder Abschnitt des mittleren Bereichs durch die Strahlungen von mindesten drei benachbart angeordneten Leuchtsegmenten (1) bestrahlt wird.

42. Bestrahlungssystem nach einem der Ansprüche 38-41,
**dadurch gekennzeichnet,**
**dass** das Bestrahlungssystem zur Aushärtung der Oberfläche eines im mittleren Bereich des Bestrahlungssystems angeordneten Mediums (9) ausgestaltet ist.

43. Bestrahlungssystem nach einem der Ansprüche 38-41,
**dadurch gekennzeichnet,**
**dass** das Bestrahlungssystem zur Bestrahlung eines im mittleren Bereich des Bestrahlungssystems angeordneten Behälter (9) aus einem strahlungsdurchlässigen Material ausgestaltet ist, wobei der Behälter zu bestrahlendes biologisches Material beinhaltet.

## Claims

1. Irradiation device,
with at least two luminous segment supports (12), each luminous segment support having a flat luminous segment (1-4) with a multiplicity of uniformly arranged luminous means (6), the luminous segment supports (12) being arranged at an angle to one another,
**characterised in that**
the emission angle of the individual luminous means (6) is at most 30°, so that directed and, with respect to the surface of the luminous segment, essentially perpendicularly emitted radiation is generated by each luminous segment (1-4), and
the luminous segment supports (12) are arranged at an angle to one another so that, when the irradiation device is arranged at a particular distance (D) from an irradiation surface (A), the radiations emitted essentially perpendicularly by the individual luminous segments (1-4) overlap essentially fully on this irradiation surface (A) such that each portion of the irradiation surface is irradiated by the overlapping radiation of at least two luminous segments.

2. Irradiation device according to Claim 1,
**characterised in that**
the luminous means (6) are arranged uniformly in columns and rows on the individual luminous segments (1-4).

3. Irradiation device according to Claim 1,
**characterised in that**
the luminous means (6) in the individual luminous segments (1-4) are arranged in rows which are alternately offset with respect to one another.

4. Irradiation device according to any one of the preceding claims,
**characterised in that**
a power supply unit is provided for supplying the individual luminous means (6) of the luminous segments (1-4) with constant current.

5. Irradiation device according to Claim 4 and Claim 2 or 3,
**characterised in that**
the luminous means (6) respectively arranged in a row are connected in series and connected to the power supply unit.

6. Irradiation device according to any one of the preceding claims,
**characterised in that**
the luminous means (6) are light-emitting diodes.

7. Irradiation device according to any one of Claims 1-5,
**characterised in that**
the luminous means (6) are laser diodes.

8. Irradiation device according to any one of the preceding claims,
**characterised in that**
the luminous segments (1-4) respectively comprise a plurality of groups of uniformly arranged luminous means (6), the luminous means (6) within a group generating radiations in an essentially equal wavelength range and the individual groups of luminous means (6) generating radiations in different wavelength ranges.

9. Irradiation device according to any one of the preceding claims,
**characterised in that**
the individual luminous segments (1-4) generate directed radiation in the ultraviolet wavelength range.

10. Irradiation device according to any one of the preceding claims,
**characterised in that**
the individual luminous segments (1-4) generate directed radiation in the infrared wavelength range.

11. Irradiation device according to any one of the preceding claims,
**characterised in that**
the individual luminous segments (1-4) generate directed radiation in the visible wavelength range.

12. Irradiation device according to any one of the preceding claims,
**characterised in that**
a base surface of the individual luminous segments (1-4) is selected so that the radiations generated by the individual luminous segments (1-4) overlap essentially fully on a rectangular irradiation surface (A).

13. Irradiation device according to any one of Claims 1-11,
**characterised in that**
a base surface of the individual luminous segments (1-4) is selected so that the radiations generated by the individual luminous segments (1-4) overlap essentially fully on a polygonal irradiation surface (A).

14. Irradiation device according to any one of Claims 1-11,
**characterised in that**
a base surface of the individual luminous segments (1-4) is selected so that the radiations generated by the individual luminous segments (1-4) overlap essentially fully on a round irradiation surface (A).

15. Irradiation device according to any one of the preceding claims,
**characterised in that**
the individual luminous segment supports with the corresponding luminous segments (1-4) are arranged at an angle to one another and next to one another.

16. Irradiation device according to Claim 15,
**characterised in that**
the luminous segment supports with the corresponding luminous segments (1-4) are arranged next to one another so that an essentially continuous emission surface is formed by the individual luminous segments (1-4).

17. Irradiation device according to any one of the preceding claims,
**characterised in that**
the luminous segment supports with the luminous segments (1-4) are fitted in a portable housing (5).

18. Irradiation device according to any one of the preceding claims,
**characterised in that**
the individual luminous segments (1-4) are arranged aligned with one another so that mid-axes of the individual luminous segments (1-4) extend in a common plane.

19. Irradiation device according to any one of the preceding claims,
**characterised in that**
the irradiation device comprises at least three luminous segments (1-4) arranged at an angle to one another.

20. Irradiation device according to any one of the preceding claims,
**characterised in that**
the irradiation device comprises a first group of luminous segments (1-3) arranged at an angle to one another and a second group of luminous segments (1-3) arranged at an angle to one another, the two groups of luminous segments (1-3) being arranged in a cross-sectional shape so that a particular luminous segment (1) is both part of the first group and part of the second group.

21. Irradiation device according to any one of the preceding claims,
**characterised in that**
the irradiation device comprises at least four luminous segments (1-4).

22. Irradiation device according to any one of the preceding claims,
**characterised in that**
the irradiation device comprises at least five luminous segments (1-4) arranged at an angle to one another.

23. Irradiation device according to any one of the preceding claims,
**characterised in that**
the irradiation device comprises at least seven luminous segments (1-4) arranged at an angle to one another.

24. Irradiation device according to any one of the preceding claims,
**characterised in that**
the irradiation device comprises a detector unit (10, 11) for recording fluorescent radiation (C) stimulated on the irradiation surface (A) as a result of the irradiation by the radiations of the luminous segments (1-4).

25. Irradiation device according to Claim 24,
**characterised in that**
the detector unit comprises an optical filter (11) with a transmission range corresponding to the stimulated fluorescent radiation (C).

26. Irradiation device according to Claim 24 or 25,
**characterised in that**
the irradiation device is part of a medical photodynamic diagnosis system.

27. Irradiation device according to any one of the preceding claims,
**characterised in that**
the individual luminous segments are arranged essentially semicircularly in cross section.

28. Irradiation device according to Claim 27,
**characterised in that**
the individual luminous segments (1-4) are arranged essentially hemicylindrically.

29. Irradiation device according to Claim 28,
**characterised in that**
a plurality of groups of luminous segments (1-4) arranged at an angle to one another are arranged essentially parallel to one another, so that luminous segments (1-4) of the individual groups are arranged essentially hemicylindrically with the radiation being directed into the interior of the hemicylinder.

30. Irradiation device according to Claim 29,
**characterised in that**
the individual luminous segment groups are directly adjacent to one another.

31. Irradiation system,
with two mutually opposite irradiation devices according to any one of Claims 28-30, the irradiation surface (A) of the irradiation devices being arranged between the two irradiation devices.

32. Irradiation system according to Claim 31,
**characterised in that**
the irradiation system is designed for whole body irradiation of a living being (P) to be positioned in the vicinity of the irradiation surface (A).

33. Irradiation system with an irradiation device according to any one of Claims 1-30 for irraddiating a body section of a living being.

34. Irradiation system according to Claim 33,
**characterised in that**
the irradiation device is fitted in a mobile housing (5) for positioning the irradiation device over the intended body section.

35. Irradiation system according to any one of Claims 31-34,
**characterised in that**
the irradiation device is part of a medical photodynamic therapy system.

36. Irradiation system with an irradiation device according to any one of Claims 1-30 for cosmetic treatment of a living being.

37. Irradiation system,
with an irradiation device according to any one of Claims 1-30 for coupling the overlapping radiation striking the irradiation surface (A) into an optical waveguide (8).

38. Irradiation system with an irradiation device according to any one of Claims 1-26,
the irradiation device comprising a multiplicity of luminous segments (1) which are arranged at an angle to one another, and in overall that they form an essentially circular cross-sectional shape, so that the radiations generated by the individual luminous segments (1) overlap in a central region of this circular cross-sectional shape.

39. Irradiation system according to Claim 38,
**characterised in that**
the radiations generated by the individual luminous segments (1) of the irradiation device have a diameter which corresponds essentially to the diameter of the central region in which these radiations overlap.

40. Irradiation system according to Claim 38,
**characterised in that**
the radiations generated by the individual luminous segments (1) of the irradiation device have a diameter which is less than the diameter of the central region in which the radiations overlap, so that each section of this central region is irradiated uniformly by the radiations of at least two luminous segments (1).

41. Irradiation system according to Claim 40,
**characterised in that**
each section of the central region is irradiated by the radiations of at least three luminous segments (1) arranged next to one another.

42. Irradiation system according to any one of Claims 38-41,
**characterised in that**
the irradiation system is designed for curing the surface of a medium (9) arranged in the central region of the irradiation system.

43. Irradiation system according to any one of Claims 38-41,
**characterised in that**
the irradiation system is designed for irradiating a container (9) made of a radiation-transmissive material arranged in the central region of the irradiation system, the container containing biological material to be irradiated.

## Revendications

1. Dispositif d'irradiation,
avec au moins deux supports de segment lumineux (12), chaque support de segment lumineux comprenant un segment lumineux plan (1-4) avec une multitude de moyens lumineux (6) disposés régulièrement, les supports de segment lumineux (12) étant disposés de façon angulaire l'un par rapport à l'autre,
**caractérisé en ce que** :
l'angle d'émission des différents moyens lumineux (6) est au maximum de 30° de telle sorte que chaque segment lumineux (1-4) génère un rayonnement dirigé et pour l'essentiel émis perpendiculairement par rapport à la surface du segment lumineux, et
les supports de segment lumineux (12) sont disposés dans un angle l'un par rapport à l'autre tel que les rayonnements émis par les différents segments lumineux (1-4) pour l'essentiel perpendiculairement se chevauchent pour l'essentiel complètement sur la surface d'irradiation (A) lorsque le dispositif d'irradiation est disposé à une distance déterminée (D) de cette surface d'irradiation (A), de telle sorte que chaque partie de la surface d'irradiation soit irradiée par le rayonnement chevauchant d'au moins deux segments lumineux.

2. Dispositif d'irradiation selon la revendication 1,
**caractérisé en ce que** les moyens lumineux (6) sont disposés sur les différents segments lumineux (1-4) de façon régulière en colonnes et rangées.

3. Dispositif d'irradiation selon la revendication 1,
**caractérisé en ce que** les moyens lumineux (6) sont disposés dans les différents segments lumineux (1-4) en rangées décalées en alternance les unes par rapport aux autres.

4. Dispositif d'irradiation selon l'une quelconque des revendications précédentes,
**caractérisé en ce qu'**une installation d'alimentation en énergie est prévue pour alimenter en courant constant les différents moyens lumineux (6) des segments lumineux (1-4).

5. Dispositif d'irradiation selon la revendication 4 et les revendications 2 ou 3,
**caractérisé en ce que** les moyens lumineux (6) disposés respectivement dans une rangée sont montés et reliés à l'installation d'alimentation en énergie en série.

6. Dispositif d'irradiation selon l'une quelconque des revendications précédentes,
**caractérisé en ce que** les moyens lumineux (6) sont des diodes électroluminescentes.

7. Dispositif d'irradiation selon l'une quelconque des revendications 2 à 6,
**caractérisé en ce que** les moyens lumineux (6) sont des diodes laser.

8. Dispositif d'irradiation selon l'une quelconque des revendications précédentes,
**caractérisé en ce que** les segments lumineux (1-4) comprennent respectivement plusieurs groupes de moyens lumineux (6) disposés régulièrement, les moyens lumineux (6) à l'intérieur d'un groupe générant des rayonnements dans une plage de longueurs d'onde pour l'essentiel identiques et les différents groupes de moyens lumineux (6) des rayonnements dans des plages de longueurs d'onde différentes.

9. Dispositif d'irradiation selon l'une quelconque des revendications précédentes,
**caractérisé en ce que** les différents segments lumineux (1-4) génèrent un rayonnement dirigé dans la plage de longueurs d'onde ultraviolette.

10. Dispositif d'irradiation selon l'une quelconque des revendications,
**caractérisé en ce que** les différents segments lumineux (1-4) génèrent un rayonnement dirigé dans la plage de longueurs d'onde infrarouge.

11. Dispositif d'irradiation selon l'une quelconque des revendications précédentes,
**caractérisé en ce que** les différents segments lumineux (1-4) génèrent un rayonnement dirigé dans la plage de longueurs d'onde visible.

12. Dispositif d'irradiation selon l'une quelconque des revendications précédentes,
**caractérisé en ce que** la surface de base des différents segments lumineux (1-4) est choisie de telle sorte que les rayonnements générés par les différents segments lumineux (1-4) se chevauchent pour l'essentiel complètement sur une surface d'irradiation (A) rectangulaire.

13. Dispositif d'irradiation selon l'une quelconque des revendications 1 à 11,
**caractérisé en ce que** la surface de base des différents segments lumineux (1-4) est choisie de telle sorte que les rayonnements générés par les différents segments lumineux (1-4) se chevauchent pour l'essentiel complètement sur une surface d'irradiation (A) polygonale.

14. Dispositif d'irradiation selon l'une quelconque des revendications 1 à 11,
**caractérisé en ce que** la surface de base des différents segments lumineux (1-4) est choisie de telle sorte que les rayonnements générés par les différents segments lumineux (1-4) se chevauchent pour l'essentiel complètement sur une surface d'irradiation (A) ronde.

15. Dispositif d'irradiation selon l'une quelconque des revendications précédentes,
**caractérisé en ce que** les différents supports de segment lumineux avec les segments lumineux (1-4) correspondants sont disposés de façon angulaire et voisine les uns par rapport aux autres.

16. Dispositif d'irradiation selon la revendication 15,
**caractérisé en ce que** les supports de segment lumineux avec les segments lumineux (1-4) correspondants sont disposés de façon voisine de telle sorte que les différents segments lumineux (1-4) forment une surface d'émission pour l'essentiel ininterrompue.

17. Dispositif d'irradiation selon l'une quelconque des revendications précédentes,
**caractérisé en ce que** les supports de segment lumineux sont logés avec les segments lumineux (1-4) dans un boîtier portable (5).

18. Dispositif d'irradiation selon l'une quelconque des revendications précédentes,
**caractérisé en ce que** les différents segments lumineux (1-4) sont disposés orientés les uns par rapport aux autres de telle manière que des axes médians des différents segments lumineux (1-4) s'étendent dans un même plan.

19. Dispositif d'irradiation selon l'une quelconque des revendications précédentes,
**caractérisé en ce que** le dispositif d'irradiation comprend au moins trois segments lumineux (1-4) disposés de façon angulaire les uns par rapport aux autres.

20. Dispositif d'irradiation selon l'une quelconque des revendications précédentes,
**caractérisé en ce que** le dispositif d'irradiation comprend un premier groupe de segments lumineux (1-3) disposés de façon angulaire les uns par rapport aux autres et un deuxième groupe de segments lumineux (1-3) disposés de façon angulaire les uns par rapport aux autres, les deux groupes de segments lumineux (1-3) disposés de façon angulaire les uns par rapport aux autres étant disposés en forme de croix de telle sorte qu'un segment lumineux déterminé (1) fasse partie aussi bien du premier que du deuxième groupe.

21. Dispositif d'irradiation selon l'une quelconque des revendications précédentes,
**caractérisé en ce que** le dispositif d'irradiation comprend au moins quatre segments lumineux (1-4).

22. Dispositif d'irradiation selon l'une quelconque des revendications précédentes,
**caractérisé en ce que** le dispositif d'irradiation comprend au moins cinq segments lumineux (1-4) disposés de façon angulaire les uns par rapport aux autres.

23. Dispositif d'irradiation selon l'une quelconque des revendications précédentes,
**caractérisé en ce que** le dispositif d'irradiation comprend au moins sept segments lumineux (1-4) disposés de façon angulaire les uns par rapport aux autres.

24. Dispositif d'irradiation selon l'une quelconque des revendications précédentes,
**caractérisé en ce que** le dispositif d'irradiation comprend un système capteur (10, 11) pour détecter un rayonnement fluorescent (C) généré sur la surface d'irradiation (A) suite à l'irradiation par les rayonnements des segments lumineux (1-4).

25. Dispositif d'irradiation selon la revendication 24,
**caractérisé en ce que** le système capteur comprend un filtre optique (11) avec une bande passante correspondant au rayonnement fluorescent (C) généré.

26. Dispositif d'irradiation selon la revendication 24 ou 25,
**caractérisé en ce que** le dispositif d'irradiation fait partie d'un système de diagnostic photodynamique médical.

27. Dispositif d'irradiation selon l'une quelconque des revendications précédentes,
**caractérisé en ce que** les différents segments lumineux sont disposés en coupe transversale pour l'essentiel en forme de demi-cercle.

28. Dispositif d'irradiation selon la revendication 27,
**caractérisé en ce que** les différents segments lumineux (1-4) sont disposés pour l'essentiel en forme de demi-cylindre.

29. Dispositif d'irradiation selon la revendication 28,
**caractérisé en ce que** plusieurs groupes de segments lumineux (1-4) disposés de façon angulaire les uns par rapport aux autres sont disposés pour l'essentiel parallèlement les uns aux autres de telle sorte que des segments lumineux (1-4) des différents groupes soient disposés pour l'essentiel en forme de demi-cylindre avec rayonnement dirigé à l'intérieur du demi-cylindre.

30. Dispositif d'irradiation selon la revendication 29,
**caractérisé en ce que** les différents groupes de segments lumineux sont directement adjacents les uns aux autres.

31. Système d'irradiation,
comprenant deux dispositifs d'irradiation selon l'une quelconque des revendications 28 à 30 situés en regard l'un de l'autre, la surface d'irradiation (A) des dispositifs d'irradiation étant disposée entre les deux dispositifs d'irradiation.

32. Système d'irradiation selon la revendication 31,
**caractérisé en ce que** le système d'irradiation est conçu pour l'irradiation du corps entier d'un être vivant (P) positionné dans la zone de la surface d'irradiation (A).

33. Système d'irradiation avec un dispositif d'irradiation selon l'une quelconque des revendications 1 à 30 pour irradier une partie de corps d'un être vivant.

34. Système d'irradiation selon la revendication 33,
**caractérisé en ce que** le dispositif d'irradiation est logé dans un boîtier mobile (5) pour positionner le dispositif d'irradiation au-dessus de la partie de corps souhaitée.

35. Système d'irradiation selon l'une quelconque des revendications 31 à 34,
**caractérisé en ce que** le système d'irradiation fait partie d'un système de thérapie photodynamique médical.

36. Système d'irradiation avec un dispositif d'irradiation selon l'une quelconque des revendications 1 à 30 pour le traitement cosmétique d'un être vivant.

37. Système d'irradiation avec un dispositif d'irradiation selon l'une quelconque des revendications 1 à 30 pour injecter le rayonnement chevauchant incident sur la surface d'irradiation (A) dans un guide d'ondes lumineuses (8).

38. Système d'irradiation avec un dispositif d'irradiation selon l'une quelconque des revendications 1 à 26,
dans lequel le dispositif d'irradiation comprend une multitude de segments lumineux (1) qui sont disposés les uns par rapport aux autres d'une telle façon angulaire qu'ils constituent une forme de coupe transversale pour l'essentiel circulaire de telle sorte que les rayonnements générés par les différents segments lumineux (1) se chevauchent dans une zone centrale de cette forme de coupe transversale circulaire.

39. Système d'irradiation selon la revendication 38,
**caractérisé en ce que** les rayonnements générés par les différents segments lumineux 1 du dispositif d'irradiation présentent un diamètre qui correspond pour l'essentiel au diamètre de la zone centrale dans laquelle ces rayonnements se chevauchent.

40. Système d'irradiation selon la revendication 38,
**caractérisé en ce que** les rayonnements générés par les différents segments lumineux (1) du dispositif d'irradiation présentent un diamètre inférieur à celui de la zone centrale dans laquelle les rayonnements se chevauchent, de telle sorte que chaque partie de cette zone centrale soit irradiée régulièrement par les rayonnements d'au moins deux segments lumineux (1).

41. Système d'irradiation selon la revendication 40,
**caractérisé en ce que** chaque partie de la zone centrale est irradiée par les rayonnements d'au moins trois segments lumineux (1) voisins.

42. Système d'irradiation selon l'une quelconque des revendications 38 à 41,
**caractérisé en ce que** le système d'irradiation est conçu pour durcir la surface d'un fluide (9) disposé dans la zone centrale du système d'irradiation.

43. Système d'irradiation selon l'une quelconque des revendications 38 à 41,
**caractérisé en ce que** le système d'irradiation est conçu pour irradier un récipient (9) en matériau perméable aux rayonnements disposé dans la zone centrale du système d'irradiation, le récipient contenant une substance biologique à irradier.
